# EUROPEAN PATENT SPECIFICATION

(11) **EP 3 291 856 B1**
(45) Date of publication and mention of the grant of the patent: **08.01.2020**
(21) Application number: 16720447.8
(22) Date of filing: 05.05.2016
(51) Int. Cl.: A61M 5/00, B65D 75/32

(54) **SEALED PACKAGES CONTAINING A MEDICAL DEVICE**
VERSIEGELTE PACKUNGEN MIT EINER MEDIZINISCHEN VORRICHTUNG
EMBALLAGES SCELLÉS CONTENANT UN DISPOSITIF MÉDICAL

(30) Priority: 05.05.2015 GB 201507696
(43) Date of publication of application: 14.03.2018
(73) Proprietor: Conceptomed AS, 8373 Ballstad (NO)
(72) Inventor: MIDE, Christian, 8373 Ballstad (NO); DANNEVIG, Anders, 4790 Lillesand (NO); ANDRESEN, Marius, 0280 Oslo (NO); BLOMVÅGNES, Rolf, 5337 Rong (NO); BRANDSNES, Ola, 0190 Oslo (NO)
(74) Representative: Dehns
(86) International application number: PCT/EP2016/060134
(87) International publication number: WO 2016/177856

(56) References cited:
- EP-A1- 2 258 302
- WO-A1-2007/091153

## Description

The present invention relates to sealed packages containing medical devices, in particular but not exclusively to sterile packages containing fluid transfer devices for use in a medical setting.

In healthcare settings, significant numbers of infections are caused by microorganism contamination due to failed aseptic technique. The clinical environment is typically populated with microorganisms that are antibiotic-resistant. Patients are particularly at risk during invasive clinical procedures and via in situ medical devices such as intravenous (IV) lines. Safe aseptic practice requires that packaging can be opened using a no-touch technique and medical devices can be easily removed from packaging without a user touching (and hence contaminating) any so-called key parts. Key parts are the critical parts of clinical equipment that come into direct or indirect contact with any liquid infusion, key sites and active key parts connected to the patient. However many packages for medical devices such as fluid transfer devices, e.g. syringes, must be pulled open carefully by a user and the onus is on the user to take hold of the device at an end away from the key parts. In practice, it is often found that medical devices are removed from packaging with the recommended no-touch technique being compromised e.g. by opening the wrong end of the packaging and accidentally touching a key part.

In addition, medical devices that carry a needle, such as syringes, must be handled with care to avoid needlestick injuries. Since the EU Council Directive 2010/32/EU on the prevention of sharps injuries in the healthcare sector, regulations have been brought into force to promote the use of "safer sharps" such as syringes with safety mechanisms that prevent a user from recapping the needle. However there remains a need for sterile packages that can protect a user from accidental needlestick injury from the moment when the package is first opened.

WO 2007/091153 discloses a packaging container comprising a housing for containing a needle assembly, along with means for opening a portion of the housing.

The present invention seeks to address or at least mitigate the problems outlined above.

According to a first aspect of the present invention there is provided a sealed package as claimed in claim 1.

It will be appreciated that such a package is quite different from a standard package where a medical device is closely contained in a cavity and a user must actively remove the device from the cavity. In a standard package the cavity is typically formed to have a shape that generally corresponds to the profile of the device, e.g. to minimise material cost and avoid unnecessary movement of the device inside the package. By providing the cavity with a fulcrum, and a pocket forward of and below the fulcrum, the device naturally tends to pivot (due to the position of its centre of mass forward of the fulcrum) and such pivotal movement can be accommodated by the pocket. This means that the device can pivot into a stable position to automatically present itself once the package is unsealed, for example by pivoting forward so that a rear end of the device becomes more accessible. There are several advantages to the device being tilted. Firstly, this can facilitate ease of access to the device with or without gloved hands. Secondly, the front end of the device being pivoted down into the front pocket can restrict the ability of users to touch key parts at the front end. For example, such a package can ensure that users do not readily come into contact with a fluid transfer tip at the front end of the device e.g. a syringe.

It will be further appreciated that the centre of mass can be predefined for any given medical device, for example depending on its dimensions. If the device is prefilled with a fluid then this may be taken into account. The cavity of the package may then be suitably dimensioned and/or shaped to provide a point of contact between the device and a wall of the cavity that defines a fulcrum, with the centre of mass of the device being positioned forward of the fulcrum. In addition, the extent of the front pocket in the cavity may be determined with reference to the size and/or shape of the device contained in the cavity. It will be appreciated that a package according to the invention may have a different cavity for any particular style of device, but of course various devices such as syringes often come in standard sizes - meaning that a package having a particular form of cavity may in practice be suitable for a number of devices e.g. from different manufacturers. Preferably the package is designed so that the fulcrum is positioned behind the centre of mass of one or more different devices, for example a range of standard syringes having volumes of 2 ml, 5 ml, 10 ml, and even 20 ml, so that the same package can be used to contain more than one device. This can also ensure that the centre of mass of any given device is positioned sufficiently forward of the fulcrum that, even if the device were to move longitudinally within the cavity (for example during transit or handling), the device will still tend to pivot forward about the fulcrum into a stable position.

While the device tends to pivot forward about the fulcrum, due to the position of its centre of mass, it may or may not be free to pivot into a stable position when contained in the sealed package. In some embodiments it is envisaged that the sealed package may allow the device to already be pivoted forward about the fulcrum, for example with the removable backing attached to the cavity so as to accommodate the device with its front end received in the front pocket. This means that once the backing is removed the device may be grasped more easily to be lifted out of the cavity, because it is presented at a tilted angle. The centre of mass being forward of the fulcrum ensures that the front end of the device tends to pivot down into the front pocket e.g. so that it is a rear end of the device which is most accessible once the cavity is unsealed. This can be particularly important for devices being unpackaged by an aseptic technique. Presenting the rear end of a device such as a syringe can help to ensure that users avoid touching a critical connection interface (such as a fluid transfer tip or needle) at the front end of the device - protecting such a key part from contamination and potentially protecting users from needlestick injury.

Where the device is already pivoted forward to present a rear end, typically the package will take up a larger volume than a package in which the device is contained in a flatter but unstable position. It may therefore be preferable for the device to be contained in the cavity in an unstable position e.g. where the device rests on the fulcrum but is substantially prevented from pivoting forward about the fulcrum. This may enable the package to be made more compact with lower material cost. Furthermore, it has been recognised that containing the device in an unstable position provides an additional advantage in that opening the package may allow the device to pivot forward so that the front end is received in the front pocket. This can act to automatically protect the front end, and preferably present a rear end of the device to the user, upon opening the package.

Removal of the backing could act in various ways to release the device from an unstable position so that it becomes free to pivot forward about the fulcrum. For example, the device could be held in an unstable position by a blocking member connected to the backing. However it is preferable for the removable backing to directly hold the device in an unstable position, so that as soon as the backing is removed the device becomes free to pivot. Thus in preferred embodiments the removable backing is arranged to substantially prevent the device from pivoting forward into a stable position. In other words, the front end of the device cannot move into the front pocket until the backing has been removed. This could be achieved, for example, by a frangible connection between the backing and the device that holds the device in an unstable position resting on the fulcrum. When the backing is removed such a frangible connection may be broken to allow the device to pivot forward so that its front end is received in the front pocket.

However a simple way to hold the device in an unstable position is for the removable backing to abut a rear end of the device so that the front end is prevented from pivoting forward about the fulcrum. For example, the cavity may take the form of an open blister with the backing attached to close the blister and form a containing wall of the package. It is therefore preferable that the device is contained in an unstable position with the removable backing forming a surface of the sealed package that is in contact with a rear end of the device. This may require the backing to be strong enough to resist the tendency of the device to pivot e.g. without tearing open under the force of the turning moment. Suitable materials for the backing may include (but are not limited to) one or more of: paper, cardboard, nonwoven webs, woven or fabric sheets, plastic films, metal foils, and laminates thereof. For example, the backing may comprise a nonwoven sheet material such as Tyvek® available from DuPont. These nonwoven materials are made from a spunbonded olefin such as HDPE to create tough, durable sheets that are stronger than paper. A nonwoven sheet material such as Tyvek® typically has a higher strength-to-weight ratio than paper, absorbs little or no moisture, and is strong and rip-resistant. The removable backing may be tested for its suitability to keep the package sealed using one or more of the standard tests for Packaging System Integrity, such as: Package Integrity (ASTM F2096: Bubble Emission Test); Seal Integrity (ASTM F1886: Visual Inspection Test, ASTM 1929: Dye Penetration Test); and/or Seal Strength (ASTM F88: Peel Strength Test, ASTM F1140: Burst/Creep Test).

A further advantage of such embodiments is that, once the backing is removed, the rear end of the device will tend to pivot beyond the previously sealed surface as the front end pivots forward. The cavity may be arranged so that this results in the rear end of the device being presented outside the cavity for ease of access. Thus in preferred embodiments the cavity is arranged such that, when the backing is removed, the device is free to pivot forward so that the rear end of the device projects out of the cavity beyond the previously sealed surface. This may be achieved by designing the geometry of the cavity relative to the fulcrum, for example the shape of the front pocket. Preferably the front pocket extends below the fulcrum to such a depth that a rear end of the device projects out of the cavity when the front end is received in the pocket. Accordingly, when the device becomes free to pivot into a stable position, the front end pivots forward/down and the rear end pivots back/up so that the rear end is in a stable position projecting out of the cavity.

In addition, or alternatively, the front pocket may have various other features of its shape and/or construction that assist a user in withdrawing the device (or part thereof) from the cavity. The front pocket in the cavity may be generally formed to match the shape of at least the front end of the device when it is pivoted forward to a stable position. This can help to ensure that the front end of the device is protected by the front pocket and can make it difficult, if not impossible, for a user to get hold of the front end - contributing to safe aseptic technique. However the front pocket must also be shaped to accommodate pivoting motion of the device into a stable position where the front end is pointing down into the pocket. Preferably the front pocket comprises a curved wall arranged in front of the device, for example a curved wall that arcs back as the pocket deepens. Such a curved wall can generally follow an arc circumscribed by the front end of the device as it pivots forward into a stable position. The front pocket having such a shape means that it can accommodate pivoting of the device while helping to minimise the volume and material cost of the cavity.

Further preferably, or alternatively, the front pocket in the cavity may provide a surface that helps to guide the device as it is withdrawn from the package. Preferably the front pocket comprises an angled wall arranged behind the front end of the device. Such an angled wall may be arranged to contact the front end of the device when it is pivoted forward into a stable position. From this position, the device can be withdrawn from the package with its front end guided along the angled wall. This can help to ensure that the front end stays inside the front pocket, where it is protected, until the device is fully withdrawn from the cavity. It is therefore preferable that a wall of the cavity in the front pocket is angled forward and down from the fulcrum to provide a surface along which the front end of the device can slide. This promotes a no-touch technique in which the device is held by a rear end as it is pulled out of the cavity.

Further preferably, or alternatively, the front pocket in the cavity may include a catch arranged to hold the front part of the device once it has pivoted forward into a stable position. This may act to hold the front part in the front pocket, where it is protected, until a user actively pulls the device with enough force to release the front part. This can help to ensure that the device does not accidentally fall out of the package once the backing has been removed and any key parts at the front end remain sterile. Furthermore, in embodiments where the front end of the device comprises a removable cover e.g. for a needle or fluid transfer tip, the Applicant has recognised that it may be desirable for the cover to be retained in the package while the device is withdrawn. This can remove the need for a user to actively uncover the front end of the device after the device has been withdrawn from the package, eliminating this step to save time, and keeping the user's hands away from the front end where key parts may be located. In some examples, the front pocket may be arranged so that the front end of the device is automatically engaged by the catch after (or at the same time as) pivoting forward to a stable position.

In some examples, user interaction may be required for the front end to become engaged by the catch, e.g. after the device has pivoted forward to a stable position. The device may need to be pushed forwards and/or rotated in order to engage the front end with the catch. This may be particularly beneficial where the device comprises, or consists of, a needle hub carrying a needle and a needle cover. While the needle hub is still in the package, tilted forward for ease of access, a user can connect another device (such as a hypodermic syringe) to the needle hub and withdraw the needle while the needle cover is held back by the catch. The action of connecting onto a tapered needle hub, for example with a Luer slip connection, may push and/or rotate the needle cover into engagement with the catch.

The catch may be any suitable means for holding the front part of the device, for example using friction or positive engagement. The catch could take the form of a gripping aperture in the front pocket, an adhesive surface, a flange arranged to engage over the front part, a recess in a wall of the cavity, or any combination of such features. The catch may be provided by a separate insert in the front pocket or a wall of the cavity in the front pocket may be formed to provide the catch. In a set of embodiments a wall of the cavity in the front pocket includes a recess to provide the catch. Such a construction can make it easier for the cavity to be formed as a single piece e.g. of moulded plastics material.

The front end of the device becoming engaged and held in the recess can act to protect the front end. It is further preferable that a wall of the cavity in the front pocket is angled forward and down towards the recess so that the front end tends to becomes engaged in the recess. This angled wall in the front pocket may enable the front end to be automatically engaged with the catch, for example the device sliding forwards under its own weight into the recess at the same time as (or after) pivoting forward to a stable position. Or the angled wall in the front pocket may enable a user to push the front end forwards into the recess, to be engaged with the catch, after the device has pivoted forward to a stable position. In embodiments where the front end comprises a removable cover, it will be appreciated that the cover may then be held by the catch while the rest of the device is withdrawn from the cavity. This may be particularly beneficial where the device comprises, or consists of, a needle hub carrying a needle and a needle cover. While the needle hub is still in the package, tilted forward for ease of access, a user can connect another device (such as a hypodermic syringe) to the needle hub and withdraw the needle while the needle cover is held back by the catch. The needle then presents itself immediately as the device is withdrawn from the package. The retained needle cover can also work as a safety bin for the used needle after use - enabling safe recapping with one hand.

The Applicant has recognised that the feature of a catch which can hold onto the removable cover of a medical device does not necessarily have to be associated with the front pocket in the cavity. The front end of the device may be withdrawn and then a catch or other gripping means located anywhere in the cavity may be used to hold the cover once a user is ready to expose the key parts of the device. Thus, at least in embodiments where the front end comprises a removable cover, it is preferable that the cavity comprises means for gripping the removable cover. As is discussed above for the catch, such a gripping means may be provided by a separate insert in the cavity, or a wall of the cavity may be formed to provide the gripping means. In a set of embodiments a wall of the cavity includes a recess to provide the gripping means. Such a construction can make it easier for the cavity to be formed as a single piece e.g. of moulded plastics material.

There is also disclosed herein for reference purposes only a sealed package containing a medical device that comprises a removable cover, the package comprising a cavity containing the device, wherein the cavity comprises means for gripping the removable cover. Such a package preferably further comprises a removable backing that seals the device inside the cavity, as in the first aspect of the invention. Furthermore, any of the other preceding features described in the context of the first aspect of the invention may equally be applied to this disclosure.

The cavity and related features described above, such as the fulcrum and front pocket, may be provided by one or more separate parts. For example, the front pocket could take the form of an insert in a larger cavity and/or the fulcrum could be provided by an insert in the cavity. However, it is preferable that the cavity is integrally formed from a single piece of material, for example a blister moulded from a plastics material. Accordingly it is the shape of the cavity that preferably forms the front pocket and fulcrum. In a preferred set of embodiments the cavity comprises a central portion having a central depth and the front pocket is defined by a forward portion of the cavity having a depth that is greater. Further preferably, the central depth may substantially correspond to the diameter of the device. It will be understood that the "diameter" of the device can be defined generally as a dimension in the depth direction, for example if the device does not have a circular cross-section or generally cylindrical shape. The central depth substantially corresponding to the diameter of the device means that the cavity does not have any excess volume, other than that provided by the front pocket to enable the device to pivot forward.

In such embodiments, the fulcrum may be formed by a corner in a wall of the cavity between the front pocket and the central portion. The angled wall of the front pocket, discussed above, may extend forward and down from this corner. While the front pocket is designed to receive a front end of the device when it pivots forward into a stable position, the central portion can be designed to receive the device in an unstable position, i.e. an unpivoted or horizontal position. In addition, or alternatively, it is preferable that the device is supported in an unstable position by a wall of the cavity in the central portion. This supporting wall of the cavity is preferably the same wall that forms a corner with the front pocket to define the fulcrum, but of course one or more separate inserts could instead provide the supporting wall in the central portion.

In some embodiments the central portion of the cavity may extend back from the front pocket to contain a rear end of the device. However, in a preferred set of embodiments the cavity further comprises a rear pocket, with the central portion positioned between the front pocket and the rear pocket. The rear pocket may have a depth that is greater than the central depth of the central portion, but preferably not as deep as the front pocket. The rear pocket may be shaped to contain a rear end of the device. In embodiments where the medical device is a fluid transfer device, such as a syringe, the rear pocket may be shaped to contain one or more features of the rear end such as a plunger and/or finger grip. When the package is placed down on a surface, the cavity therefore comprises two points of contact spaced apart by the central portion. The front and rear pockets function to support the package on the surface. Furthermore, such an arrangement of different depth pockets helps a user to visually distinguish between front and rear ends of the package, so that they can pick up the package and peel open the backing at the rear end of the device. This is another feature that promotes safe aseptic technique.

In some embodiments the central portion may be substantially flat, in other words, extending in a substantially straight line between the front pocket and the rear pocket. However the Applicant has recognised that the central portion may beneficially provide an additional pocket that can be used to contain one or more other components associated with the device, for example a cap. Thus in at least some embodiments the central portion comprises a central pocket, preferably a central pocket having a depth that is less than the depth of the front pocket. In embodiments where the cavity also includes a rear pocket, as described above, the depth of the central pocket may me more or less than the depth of the rear pocket. When the package is placed down on a surface, the cavity may therefore comprise three points of contact that function to support the package on the surface: a first point of contact provided by the front pocket, a second point of contact provided by the central pocket, and a third point of contact provided by the rear pocket. This can make the package very stable when it is placed on a horizontal surface. Stability may be enhanced by ensuring that the outer surface of the central pocket is aligned with an outer surface of the rear pocket and/or front pocket. Preferably the central pocket and the rear pocket are aligned in a common plane so as to support the package on a surface while the front pocket may be tilted. Thus in a preferred set of embodiments the central pocket comprises an outer surface extending in a plane that is aligned with an outer surface of the rear pocket.

In a preferred set of embodiments the sealed package contains a medical device and an additional component for use with the medical device, for example a fluid transfer device and a cap for use with the device e.g. a cap to cover the fluid transfer tip so that it is protected from human touch even after the device is removed from the package. An advantage of containing the medical device and an additional component together in the same package is that they can be treated with a common sterilisation operation. The additional component may be contained in a further pocket provided anywhere in the cavity, for example a further pocket formed in the front pocket (or rear pocket, where provided), or a further pocket formed in the cavity separately from the front pocket (and/or rear pocket, where provided). The additional component is preferably contained in the central pocket, and preferably the additional component is wholly contained in the central pocket so as not to physically contact the device. The central pocket may be shaped to contain, and optionally grip, the additional component. The central pocket may be formed as a continuous three-dimensional shape, for example a hemispherical or cylindrical shape. In some embodiments the central pocket may be formed as a stepped or changing shape, for example comprising a first depth around a periphery of the pocket and a second, greater, depth in a central portion of the pocket. In addition, or alternatively, the central pocket may be tapered in shape. One or more of these features may assist the central pocket in gripping a component such as a cap. One or more of these features may assist in insertion and/or removal of the component.

It has already been discussed above how a user may pick up the package and hold it in a suitable orientation to remove the backing from a rear end, furthest from the front pocket. It has been recognised that the shape of the cavity may otherwise allow the device to move back and forth in the package, e.g. to slide longitudinally along the central portion, even if it is prevented from pivoting forward into the front pocket. However this may not be desirable, in case movement of the device risks damage to its front end and/or damage to the cavity. Accordingly, in a preferred set of embodiments a wall of the cavity is formed with an internal feature that abuts the device to prevent it from sliding forwards in the cavity. In embodiments where the cavity comprises a rear pocket, this internal feature may comprise a corner between the central portion and the rear pocket. Such a package preferably comprises a corner formed between the central portion and the rear pocket that is arranged to abut the device to prevent it from sliding forwards in the cavity. A rear end of the device, for example the finger grip of a syringe, may conveniently abut this corner so that longitudinal movement is not possible. This can prevent the front end, e.g. fluid transfer tip, from hitting a front wall of the cavity, protecting the tip from damage and avoiding forces that could tear open the package. Such an internal feature may thereby ensure that the front end of the fluid transfer is spaced from a wall of the cavity that forms the front pocket.

The Applicant has recognised that the shape of the central portion in the cavity can be used to provide further benefits when containing a device, for example a hypodermic syringe or other medical device that carries surface markings. The central portion may be arranged so that its walls are not fully in contact with the device. Preferably the central portion comprises side walls that support the device and a lower wall that is spaced away from the device. The lower wall may define a central pocket as described above. Further preferably the device comprises one or more surface markings that are positioned above the lower wall of the central portion, in particular printed markings. The lower wall being spaced from the surface markings on the device means there is no contact and friction tending to rub off the markings, for example if the package is exposed to vibrations during transit.

There is also disclosed herein for reference purposes only a sealed package containing a medical device that has printed markings on a surface thereof, the package comprising a cavity containing the device, wherein the cavity comprises side walls that support the device and a lower wall that is spaced away from the surface that has the printed markings. Such a package preferably further comprises a removable backing that seals the device inside the cavity, as in the first aspect of the invention. Furthermore, any of the other preceding features described in the context of the first aspect of the invention may equally be applied to this further disclosure.

A cavity according to the invention may have one or more further features as described below. The cavity preferably has an outer profile that follows the profile of the inner walls that contain the device, to save on material, although this is not necessary in all embodiments. The outer profile of the cavity may be shaped so as to determine how the package rests on a surface. For example, it is preferable that the removable backing forms an upper surface of the package when it is placed on a surface, for ease of access. In addition, or alternatively, the cavity can be shaped so as to ensure that the device will still tend to pivot when the package rests on a surface, preferably a generally horizontal surface such as a trolley. It is therefore preferable that the cavity has an outer profile below the device which is shaped such that, when the package rests on a horizontal surface, the predefined centre of mass of the device is forward of the fulcrum.

It has already been described above how an internal feature of the cavity may be arranged to abut the device to prevent it from sliding forwards and hitting a wall of the front pocket. More generally, the cavity may comprise one or more features (in addition, or alternatively) that are arranged to grip or otherwise hold the device, preferably holding the device in an unstable position at least until the backing is removed. As mentioned above, such features may ensure that the front end of the device is spaced from a wall of the cavity that forms the front pocket. Furthermore, the cavity may be shaped to fit around a given device and thereby prevent rotation while it is contained in the package. For example, the rear pocket (where provided) may be shaped to match the finger grip at a rear end of a hypodermic syringe and this prevents the syringe from rotating. This can also help to ensure that any surface markings remain spaced away from a wall of the cavity so they cannot be rubbed off.

The removable backing preferably comprises a film that is attached to the cavity to form an upper surface of the sealed package. The film may be attached in such way that it can be peeled off easily by a user, preferably with one hand. As is discussed above, it is advantageous for a user to be able to hold the sealed package in one hand and access a rear end of the device while the front end remains protected inside the cavity. This can be assisted by the backing comprising a tab arranged at an opposite end of the cavity to the front pocket. In some embodiments the backing may be arranged such that it can only be partially removed, e.g. peeled back far enough for the rear end of the device to project out of the cavity but the backing remains attached to the cavity. In other embodiments the backing may be arranged such that it is wholly removable from the cavity. This may be particularly desirable where a user wishes to unseal the package and tip out the device onto an aseptic field (such as a procedure trolley or portable tray) using a no-touch technique. Once the backing has been removed, the sterile interior of the cavity may even be used as a micro critical aseptic field.

It has already been mentioned above that, in preferred embodiments, the cavity is integrally formed from a single piece of material, for example a blister moulded from a plastics material. Whether the cavity is formed from a plastics material or not, it is preferable for the cavity to be substantially transparent, so that a user can readily view the device contained in the package before it is unsealed. The backing may also comprise a substantially transparent material for this reason.

In order for a user to be able to grip the package in one hand while using the other hand to remove the backing and withdraw the device, and for such gripping not to interfere with the automatic pivoting of the device, the cavity is preferably formed of a relatively stiff or hard material. In other words, it is preferable that the cavity is not manually deformable. In preferred embodiments where the cavity is formed from a plastics material, preferably a thermoplastic material suitable for blow moulding or injection moulding, the plastics material may be chosen from: PET (polyethylene terephthalate); PVC (polyvinyl chloride); PCTFE (polychlorotrifluoroethylene); PP (polypropylene); PE (polyethylene); PC (polycarbonate) or copolymers of any of these.

Although it is mentioned above that the cavity is preferably not manually deformable, there may be certain circumstances where it is desirable to be able to deform one or more walls of the cavity by applying a certain degree of force. For example, as will be described in more detail below, it may be beneficial to deform one or more walls of the cavity during a manufacturing process for the package so as substantially prevent the device from pivoting forward into a stable position until the removable backing has been applied. The cavity may therefore be formed of a relatively stiff material, that resists deformation when gripped manually, but a material that can still be deformed when gripped tightly enough e.g. before the package is sealed. The cavity is preferably formed of an elastic material that helps the cavity to return to its original shape after any deformation. The thermoplastic materials listed above may be found suitable in this respect. For example, the cavity may be formed of thermoplastic sheet material (e.g. PE sheet which is thicker and stiffer than PE film). The sheet material is preferably at least 0.2 mm, 0.3 mm, 0.4 mm or 0.5 mm thick. Such thermoplastic sheet material is thicker and stiffer than conventional plastic films as conventionally used to form soft packages for devices such as syringes.

While the package has so far been described in the context of a cavity containing the device, it will be appreciated that the package may comprise one or more such cavities. In at least some embodiments the package may comprise a plurality of such cavities, each containing a medical device and/or part of a medical device and/or component for use with the medical device. For example, such a package may contain both a needle and a hypodermic syringe in parallel - both devices being presented by tilting forwards into respective front pockets for safe handling with minimal risk of needlestick injury. For example, such a package may also contain a cap for the medical device - the cap preferably being presented in an orientation ready for connection. The removable backing could cover such multiple cavities and be lifted off in a single operation, presenting multiple device or components at the same time, e.g. vials, catheters, needles, syringes, etc. A common sterilisation operation may be applied to the device and other parts or components contained in the same package.

Sealed packages according to embodiments of the present invention may contain many different types of medical device. According to the U.S. Food and Drug Administration (FDA), a medical device can be defined by section 201(h) of the Federal Food, Drug and Cosmetic Act (FFDCA) as "an instrument, apparatus, implement, machine, contrivance, implant, in vitro reagent, or other similar or related article, including a component part, or accessory which is: (1) recognized in the official National Formulary, or the United States Pharmacopoeia, or any supplement to them; or (2) intended for use in the diagnosis of disease or other conditions, or in the cure, mitigation, treatment, or prevention of disease, in man or other animals; or (3) intended to affect the structure or any function of the body of man or other animals, and which does not achieve its primary intended purposes through chemical action within or on the body of man or other animals and which is not dependent upon being metabolized for the achievement of any of its primary intended purposes." The CDRH classification database contains a list of all products considered by the FDA to be medical devices.

In a preferred set of embodiments the medical device comprises a fluid transfer device. The fluid transfer device may comprise any type of device used to transfer fluid - liquid and/or gas - either to or from a fluid receptacle. The fluid receptacle may be inanimate or it may be part of a living subject, for example a bodily cavity, organ or vessel, such as a vein or artery. For example, the fluid transfer device may comprise one or more of: a hypodermic syringe; a prefilled syringe; an autoinjector; an insulin pen injector; an implant injector; a catheter; a catheter connector or valve; an IV delivery device; a blood collection tube; a Luer connector; a fluid vial; or any related accessory. A Luer connector may be connected to, or suitable for connection to, a fluid pump, stopper, closing cone, stopcock, aspirator, suction device, hose, or tubing. The fluid transfer device may be made to meet the relevant medical standard(s), for example ISO 7886 for sterile hypodermic syringes. A hypodermic syringe may have a volume of 2 ml, 5 ml, 10 ml, or 20 ml.

In a preferred set of embodiments the front end of the fluid transfer device comprises a fluid transfer tip, without or with a needle connected to the fluid transfer tip. The fluid transfer tip may be a key part that comes into contact (directly or indirectly) with a patient, such that it is desirable for the tip to be protected by the package. In one set of embodiments the fluid transfer tip may be needleless, for example the fluid transfer tip of a prefilled flush syringe. In another set of embodiments, the fluid transfer tip may include a needle. The needle may be integrated with the tip or permanently connected thereto, for example the injection needle of a prefilled single dose pouch or the needle of an auto-injector such as an epinephrine "EpiPen" or insulin pen injector. In other embodiments the fluid transfer tip may include a needle hub separably connected thereto. Such a needle hub may be connected to the fluid transfer tip of a variety of different devices such as a hypodermic syringe or catheter connector.

A sealed package according to embodiments of the present invention may be particularly well suited to containing a fluid transfer device that comprises a disconnecting member mounted at the fluid transfer tip. In a preferred set of embodiments the front end of the fluid transfer device comprises a fluid transfer tip and a lever member pivotally mounted relative to the fluid transfer tip. The lever member may be provided to assist in disconnecting the front end from a fluid transfer connection in use, for example any of the fluid transfer devices or connections described and claimed in the Applicant's prior publications WO 2013/164358, WO 2014/020090 and WO 2015/014914. In such embodiments the cavity may be able to protect the lever member from accidental activation during transport. The weight of the lever member can ensure that the fluid transfer device quickly reaches a stable (i.e. fully tipped) position, for example after only a small amount of the backing is removed. A fluid transfer device that comprises a lever member has been found to reduce the risk of hand contamination when detaching a medical device interface, e.g. disconnection of a syringe from an IV cannula with an injection port (for administration of fluid), e.g. disconnection of a syringe from an IV port, e.g. withdrawal of a needle following fluid preparation or injection. Disconnection using a lever member significantly reduces the risk of needlestick injury, improves no-touch technique and reduces the risk of key part contamination. The lever member moves a user's hand away from proximity to the key parts, and visually reinforces protection of the key parts, at the front end. Furthermore, single-handed operation provides users with a free hand - which can be particularly helpful in anaesthetic and emergency scenarios.

The fluid transfer tip may form a Luer lock or a Luer slip connection. Preferably the fluid transfer tip is tapered to form a friction fit when connected to a corresponding hub, further preferably comprising a male connector tip that is tapered to form the friction fitting when inserted into a corresponding female hub, e.g. a needle hub or IV port. The fluid transfer tip may be tapered to provide a standard Luer Slip connection. The fluid transfer tip may optionally include a threaded collar to provide a standard Luer Lock connection. Although standard Luer Slip or Luer Lock connections use a male tapered tip that fits inside a female hub, it is envisaged that this could be reversed and the fluid transfer tip could be a female part having an internal taper to form friction fit with a corresponding male hub. Alternatively, or in addition, the fluid transfer tip may comprise means for gripping a hub in a locked position. This may include a snap-fit connection, latch means, gripping finger, etc. that positively engage i.e. grip a hub when it is connected to the tip. This may be particularly suitable for high pressure fluid connections e.g. when transferring more viscous fluids.

The fluid transfer tip may connect, in use, to one or more of: a fluid transfer hub connected to an interface for intravenous (IV) medication, i.e. an IV port, a needle for fluid preparation or administration, a cannula port, or a wing infusion set. It will be appreciated that the fluid transfer tip is received in the front pocket of the cavity when the device is free to pivot forward, this key part then being protected while a rear end of the device is presented to a user. This helps to ensure that the fluid transfer tip is kept sterile and protected from contamination. A user may easily grasp the rear end of the tilted fluid transfer device and, in embodiments where the package also contains an additional component such as a cap in a further pocket, the fluid transfer tip may be connected to the additional component in a single one-handed operation. In various embodiments the fluid transfer device is a hypodermic syringe. In such embodiments the rear end of the fluid transfer device may comprise a plunger, which is preferably received in the rear pocket (where provided).

The contents of the Applicant's prior publications WO 2013/164358, WO 2014/020090 and WO 2015/014914 are hereby incorporated by reference. In a preferred set of embodiments, the lever member comprises a front surface that is substantially transverse to the axis of the male connector tip and one or more side surfaces that extend in a direction substantially parallel to the axis of the male connector tip, the front surface being moveable along the male connector tip between a first position proximal to the fluid chamber and a second position spaced from the first position towards a distal end of the male connector tip, wherein the side surface(s) form a shroud extending back from the front surface towards the fluid chamber and thereby at least partially surround the fluid chamber. Such fluid transfer devices are able to provide single-handed operation and precise disconnection power.

In a set of embodiments a cap for the fluid transfer tip is also contained in the cavity, separately from the fluid transfer device, in a further pocket and preferably in a central pocket as described above. By providing a cap for the fluid transfer tip in the same sealed package as the fluid transfer device, aseptic procedures can be promoted as a user may insert the tip into the cap as soon as the package has been opened, thereby avoiding one or more risks of contamination that typically arise from a user touching (e.g. accidentally) the tip, or from the tip coming into contact with non-aseptic fields or surfaces in the surroundings, or from air contaminants. The cap may then protect the fluid transfer tip until such time as a user is ready to connect the tip to another component, such as a needle hub or IV port.

In a set of embodiments a needle hub is connected to the fluid transfer tip. The needle hub preferably comprises a needle and a removable needle cover. A lever member as described above may be provided to assist in disconnecting the needle hub from the front end of the fluid transfer device. In other embodiments, the package may contain a sheathed needle alone. In such embodiments the medical device may consist of a needle hub, carrying a needle, and a needle cover. The needle cover is preferably a removable cover that can be gripped by the cavity, for example by a dedicated recess provided in the cavity, to assist in removing or replacing the cover as described above.

In many, if not all, embodiments of the present invention, the sealed package preferably comprises a sterile package. It will be understood that a sterile package is a package intended to maintain the sterility of terminally sterilised medical devices until the point of use. A sterile package may be defined as one that meets the requirements of International Standard ISO 11607 and/or the BS EN 868 series of European standards.

In the description above, when the medical device is said to be in a stable position, this is considered to be the natural position of the device were it to be placed in the cavity with no other forces acting on the device or the cavity so as to counteract the force of gravity acting through the predefined centre of mass to effect pivoting about the fulcrum. The device is considered to be in an unstable position when it is moved out of its stable position by one or more forces acting to counteract the force of gravity and prevent the device from pivoting freely about the fulcrum. In preferred embodiments described above, the device may be held in an unstable position by the backing that seals the device inside the cavity. However, at any point in time there may be alternative or additional forces acting on the package so as to substantially prevent the device from pivoting forward into a stable position.

During manufacture of the sealed package, typically a medical device would be placed into the cavity of the package before applying the removable backing. Due to the position of front pocket and the fulcrum, this would typically allow a front end of the medical device to pivot forward within the cavity. Depending on the length of the device relative to the depth of the cavity, such pivoting may cause a rear end of the device to project out of the cavity and this could interfere with application of the backing. For example, in embodiments where the medical device comprises a syringe, the plunger of the syringe may protrude from the cavity. It is generally desirable to minimise the depth of the cavity so as to reduce material cost for the package. It may be desirable to prevent the medical device from pivoting in the cavity at least during the manufacturing step of sealing the package.

Therefore in a preferred set of embodiments the package further comprises a support arranged outside the package to deform the cavity so as to substantially prevent the device from pivoting forward into a stable position inside the package. This is advantageous as the support may hold the device horizontally within the cavity so that the backing can be applied without any obstruction from the pivoted device. This ensures that the backing can be tightly sealed to the cavity of the package, which is advantageous over a loose backing which may permit the device to move undesirably within the package.

Such a support may be substantially rigid, for example to ensure that sufficient force is applied to deform the cavity. However it may be difficult to apply a rigid support to the outside of the package. Thus, in some embodiments, such a support may be flexible, e.g. springy, so as to apply a resilient force to deform the cavity. For example, the support may comprise a flexible clip e.g. to assist in attachment of the support to the outside of the package. The support may take the form of a generally U-shaped clip. The support may be made from an elastic metal or plastics material.

In a further preferred set of embodiments the support is arranged to deform one or more walls of the front pocket. Deforming the walls of the front pocket reduces the volume of the front pocket and prevents the medical device from pivoting into the pocket. By deforming the walls of the front pocket the support can also be advantageously positioned such that it does not interfere during application of the backing to the cavity. Once the backing has been applied to seal the package, it may be desirable to remove the support. Acordingly the support is preferably removable. For example, the support may be applied to the package before attaching the backing, then removed after the sealing process is complete and before the package is shipped or supplied to a user.

It will be appreciated that deformation of the cavity achieved by the support is preferably a temporary or elastic deformation so that the cavity automatically returns to its intended shape and form once the support is removed. Accordingly the support is constructed so as not to cause a plastic deformation of the cavity. Preferably the cavity returns to its original shape when the support is removed. The contained device is therefore only prevented from pivoting forward during the time that the support is applied to the outside of the package. As is mentioned above, such a temporary support may be particularly useful when the package is being sealed. This is considered novel and inventive in its own right.

According to a further aspect of the invention there is provided a method of sealing a package containing a medical device as claimed in claim 14.

According to such a method the medical device is supported such that it cannot pivot forward about the fulcrum into a stable position and hence the front end is not received in the front pocket and a rear end of the device does not tend to protrude out of the cavity and interfere with application of the backing. As is described above, deforming the cavity preferably comprises deforming one or more walls of the front packet. Preferably the deformation is elastic. In such a method, the step of deforming the cavity may take place before or after containing the medical device in the package.

In preferred embodiments, the step of deforming one or more walls of the cavity comprises arranging a support outside the package. A suitable support may have any of the features described above.

The method may further comprise applying the removable backing so as to substantially prevent the device from pivoting forward into a stable position inside the package. This means that, once the backing is applied, the support is no longer needed to hold the device in an unstable position. Accordingly the method may further comprise releasing the one or more walls of the cavity such that the medical device is no longer supported by the cavity in an unstable position. However, as is mentioned above, the device may instead be supported in an unstable position by the backing. The releasing step preferably takes place after applying the removable backing. The wall(s) of the cavity may be released, for example, by removing a support from the outside of the package. This removes the external forces that preferably cause temporary or elastic deformation of the cavity. The cavity is then free to return to its original shape. Ideally the cavity is elastic so that it can return to its original shape automatically. However it is also envisaged that the method may further comprise reforming the cavity after releasing the wall(s), for example to ensure that the front pocket and fulcrum are not compromised by the steps used to seal the package.

Some preferred embodiments of the present invention will now be described, by way of example only, and with reference to the accompanying drawings, in which:
Fig. 1 shows an embodiment of a packaging containing a syringe;
Fig. 2 shows an embodiment of the packaging where the backing has been partially removed;
Fig. 3 shows a perspective view of the packaging;
Fig. 4 shows a side profile of the packaging;
Fig. 5 shows a view from the underside of the packaging;
Fig. 6 shows an end-on view of the packaging;
Fig. 7 shows a side view of the packaging, highlighting the front pocket;
Fig. 8 shows a side profile of the packaging, highlighting a slice through relating to the antivibration feature;
Fig. 9 shows a cross-sectional view through line A-A in Figure 8;
Fig. 10 shows a perspective view of the packaging containing a syringe;
Fig. 11 shows a side view of the packaging, drawing attention to the internal structure which prevents the syringe from sliding within the packaging;
Figs. 12a-c show the backing being removed from the package and the syringe pivoting;
Figs. 13a-c show a user removing the syringe from the package by physically pulling the syringe;
Figs. 14a-c show a user removing the syringe by tipping the package over such that it falls out onto a surface;
Figs. 15a-b show another embodiment of a packaging cavity for a needle - an isometric view in

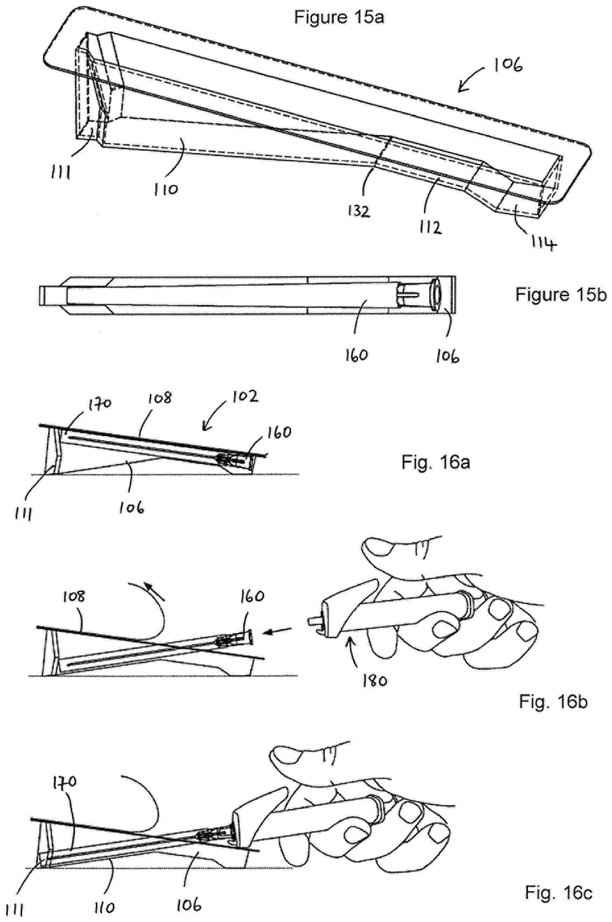

Fig. 15a of the empty cavity and a top view in Fig. 15b of the cavity containing a needle hub;

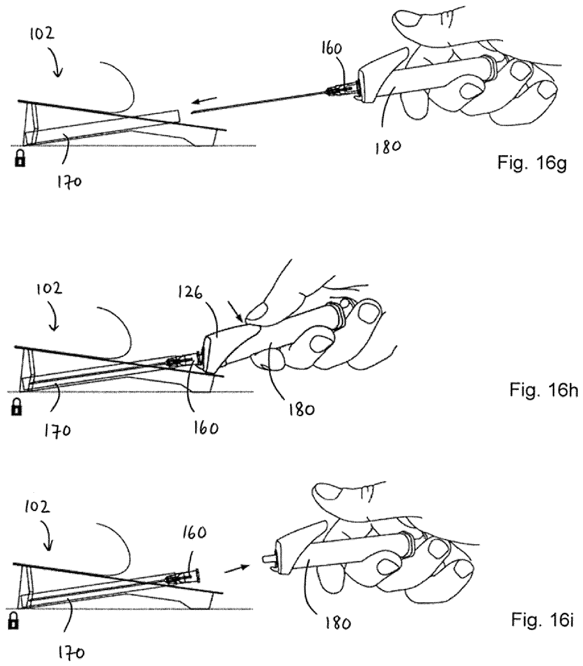

Figs. 16a-i show a user opening such a package, attaching a syringe to the needle, optionally fastening the needle, and then returning the needle after use;

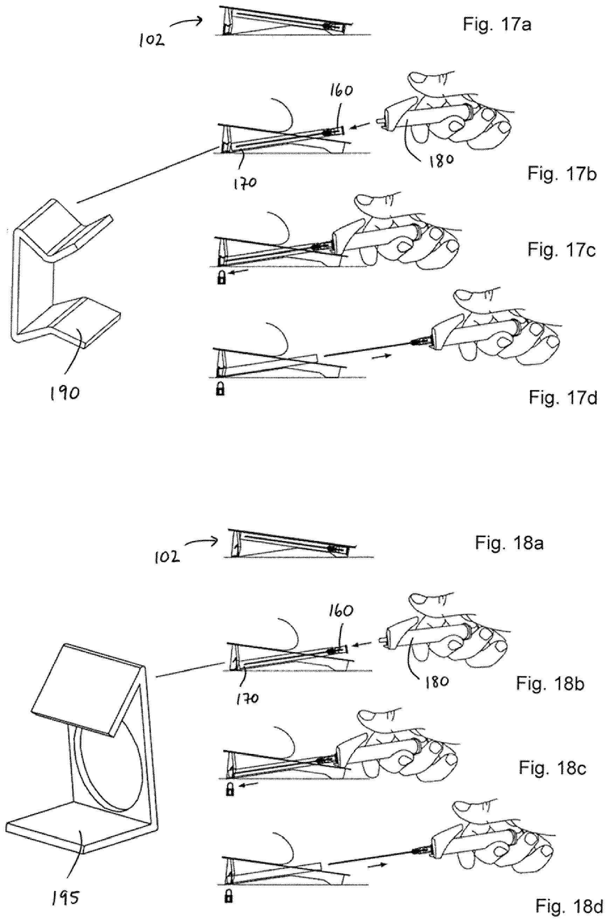

Fig. 17 shows another embodiment of a package containing a needle;
Fig. 18 shows another embodiment of a package containing a needle;

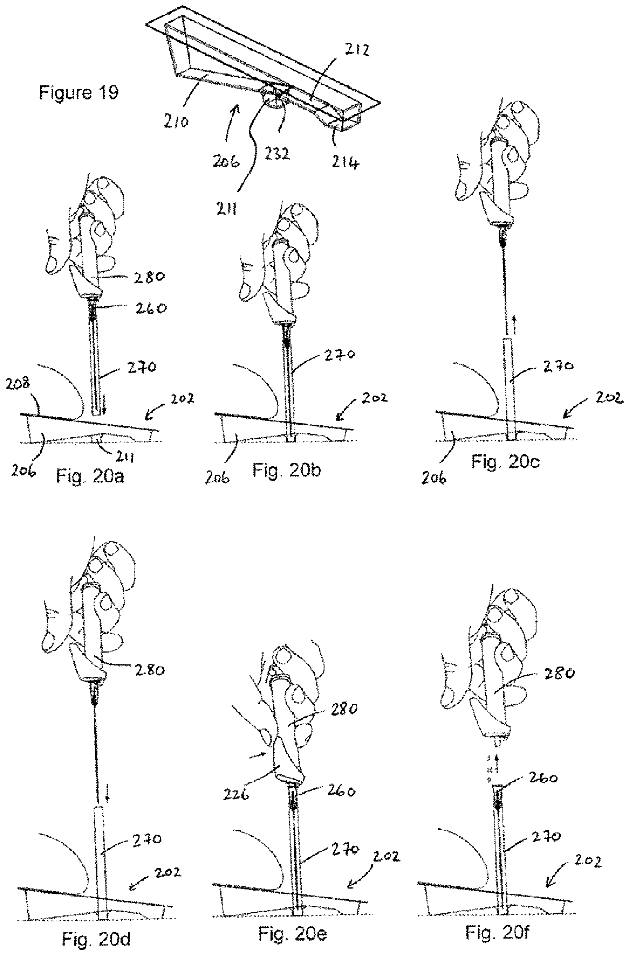

Fig. 19 shows another embodiment of a packaging cavity for a needle;
Figs. 20a-f show a user attaching a syringe to the needle, withdrawing the needle from its cover, and then returning the needle after use;

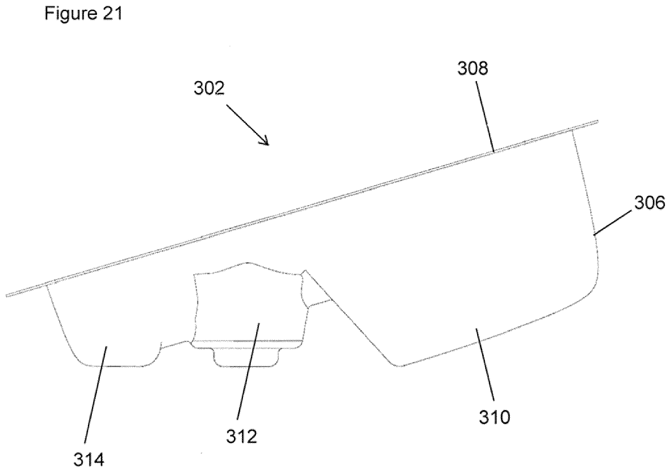

Fig 21 shows another embodiment of a package;

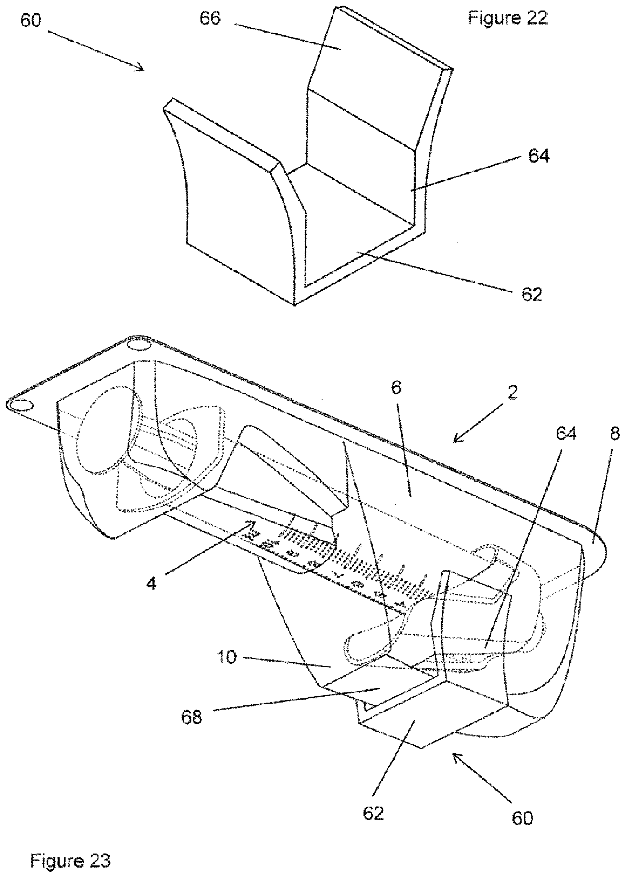

Fig. 22 shows a U-shaped clip that can be used as a support for a package;

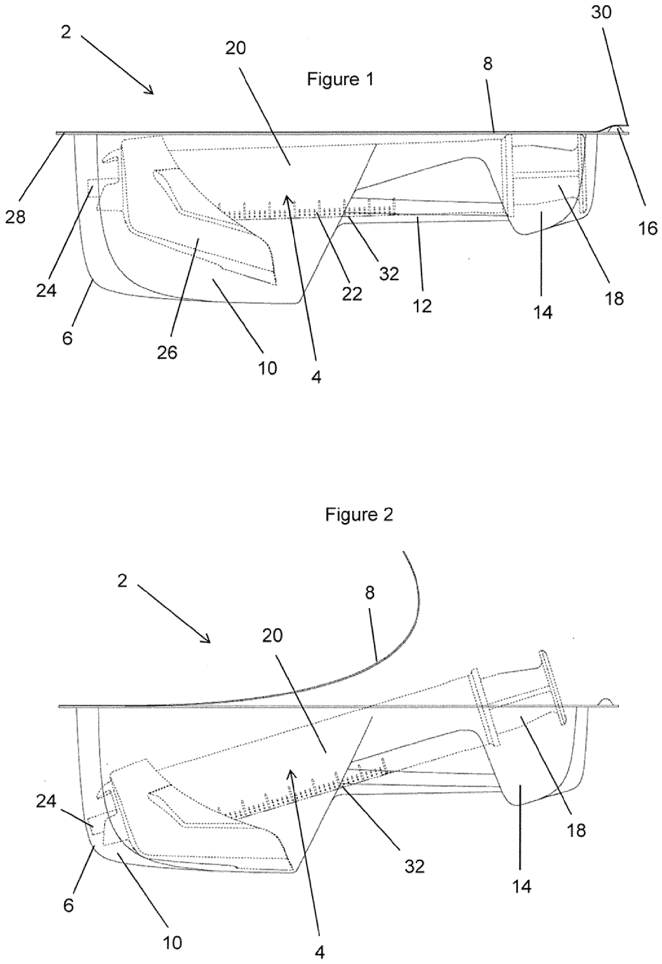

Fig. 23 shows a perspective view of the U-shaped clip of Fig. 22 applied to the package of Figs. 1-14;

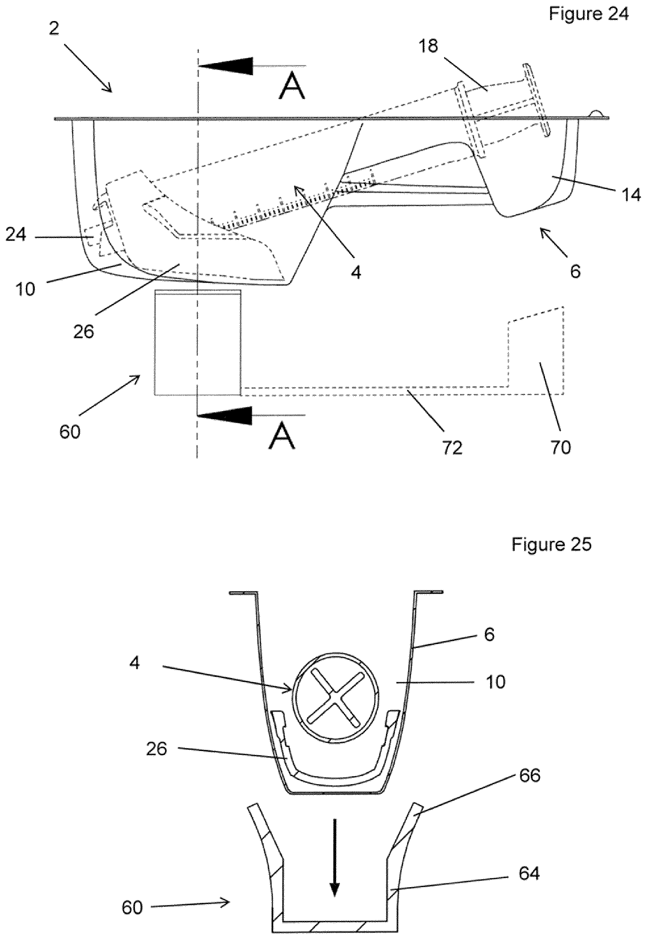

Fig. 24 shows the U-shaped clip being applied;
Fig. 25 shows the package being pressed down into the U-shaped clip;

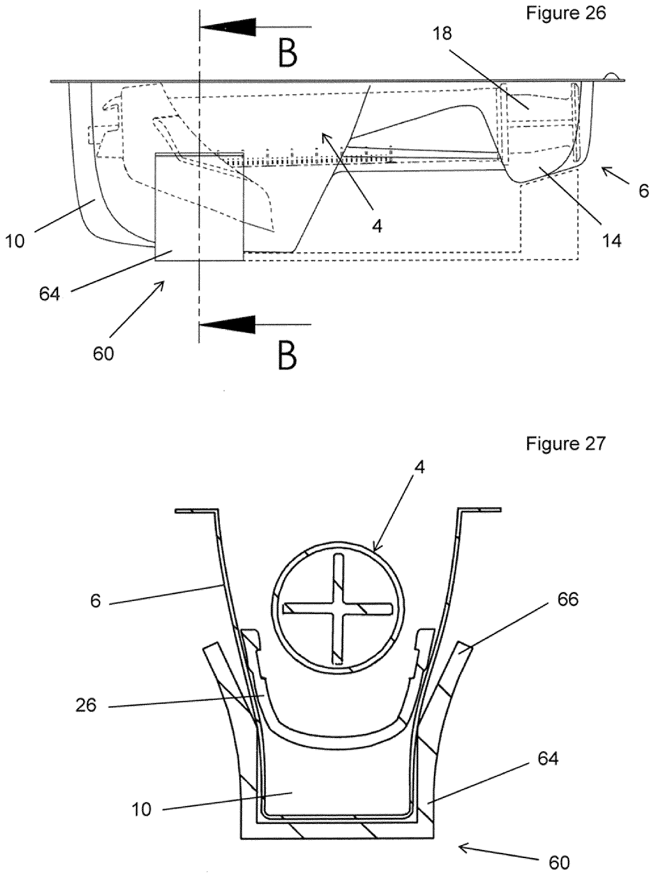

Fig. 26 shows a side cross-sectional view of the U-shaped clip applied to the package; and
Fig. 27 shows a front cross-sectional view of the U-shaped clip applied to the package.

Figure 1 shows an embodiment of a package 2 containing a typical hypodermic syringe 4. The package 2 is comprised of a cavity 6 and a backing 8. A front pocket 10 is located at one end of the cavity 6 and a central flat portion 12 extends from the front pocket 10 towards a rear pocket 14 at the opposite end of the cavity 6. The cavity 6 is moulded from a relatively hard, clear thermoplastic material, for example PET (polyethylene terephthalate) or a PET copolymer such as PETG. The cavity 6 holds it shape because it is not made from a plastics film, like traditional syringe packages, but sheet material such as 0.4 mm thick PET. This ensures that the cavity 6 does not easily deform when handled by a user. The backing 8 consists of a nonwoven sheet material such as Tyvek® (available from DuPont). A 0.15 mm thick sheet of Tyvek® has been found tough enough to avoid the syringe 4 piercing through the backing 8 e.g. during transit.

Also shown in Figure 1 is a protrusion 16 moulded on the top surface of the cavity 6. The backing 8 is sealed to the top surface of the cavity 6 around a peripheral portion 28 such that when it is removed there is an opening to allow the syringe 4 to be removed. The backing 8 is not sealed past the point of the protrusion 16. The protrusion 16 therefore acts to hold an end portion 30 of the backing 8 away from the cavity 6 so that it forms a tab which can easily be grabbed by a user. This is particularly advantageous when the package 2 is used in medical situations where a user may be wearing sterile gloves, which can reduce manual dexterity and make opening the packaging 2 difficult.

The syringe 4 contained in the package 2 comprises a plunger 18, a barrel 20 with scale markings 22, a fluid transfer tip 24 and a lever member 26 which is mounted around the fluid transfer tip 24. It can be seen that the front pocket 10 has a depth which is greater than that of the central portion 12. It can be seen that the syringe 4 pivots about a fulcrum 32 formed where the surface of the barrel 20 comes into contact with a corner in the cavity wall where the central portion 12 drops down into the front pocket 10 of the cavity 6. In the embodiment shown, as the lever member 26 is positioned around the fluid transfer tip 24, due to the mass this adds in the region proximal to the fluid transfer tip 24 it is evident that the centre of mass is forward of the fulcrum 32.

As seen in Figure 2, the backing 8 can be partially removed from the cavity 6 such that the syringe 4 is free to pivot within the cavity 6. The syringe 4 pivots such that the plunger 18 protrudes from the cavity 6 and the fluid transfer tip 24 moves towards the bottom of the cavity 6 into the front pocket 10. This helps to reduce the risk of contamination of the fluid transfer tip 24, firstly because the plunger 18 is readily accessible meaning that the user is more likely to grab the syringe 4 by the plunger 18, and secondly because the fluid transfer tip 24 moves towards the base of the cavity 6 making it a less attractive portion to remove the syringe 4 by.

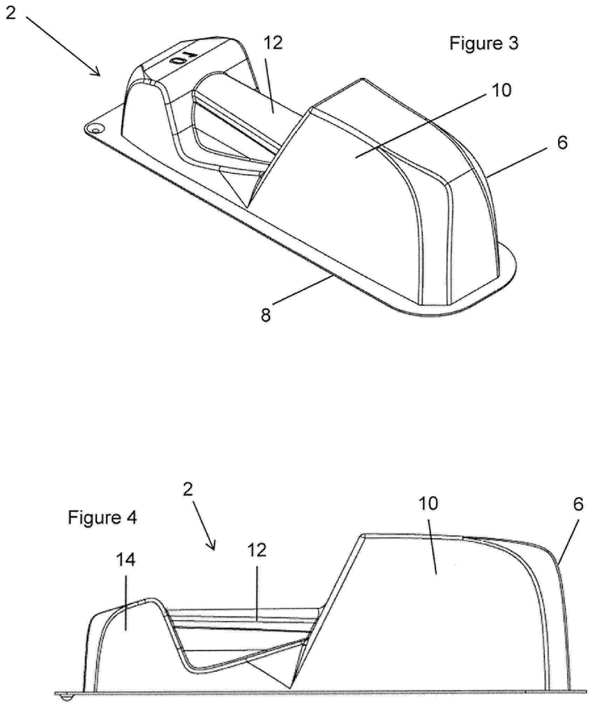

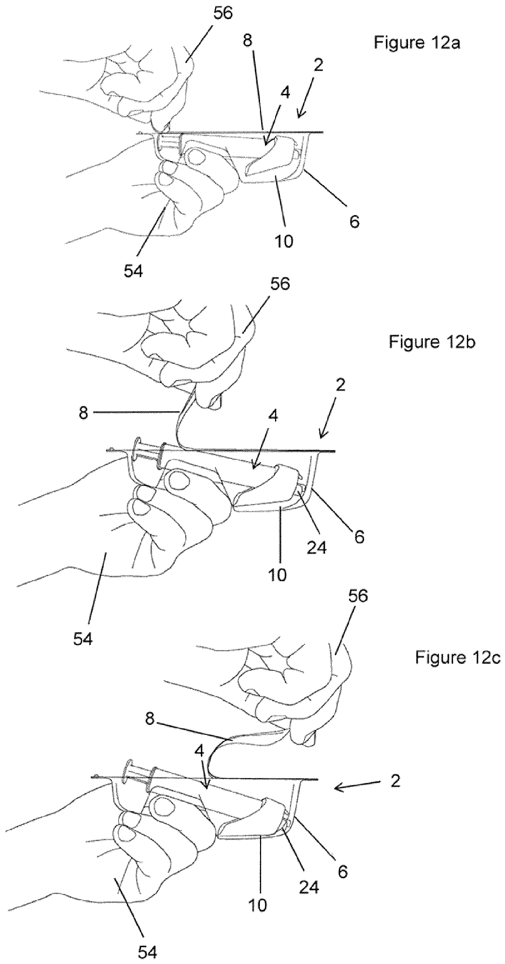

Figure 3 shows a different perspective of the packaging 2. It can be seen that the central portion 12 where the syringe barrel 20 rests is narrower than the front pocket 10 of the cavity 6. The outer surface of this narrower central portion 12 provides a surface for the user to grab the cavity 6 when removing the backing 8 and the syringe 2. This can be seen in more detail in later Figures, specifically: Fig. 12, 13 and 14. This narrower central portion 12 also fits around the barrel 20 on the syringe 4 to restrict its movement within the packaging 2.

Figure 4 shows a side view of the package 2. In this Figure it can be seen that the front pocket 10 extends substantially deeper that the flat section 12 to enable the syringe 4 to pivot within the cavity 6. The extra volume defined by the front pocket 10 provides the space for the fluid transfer tip 24 to pivot forward into.

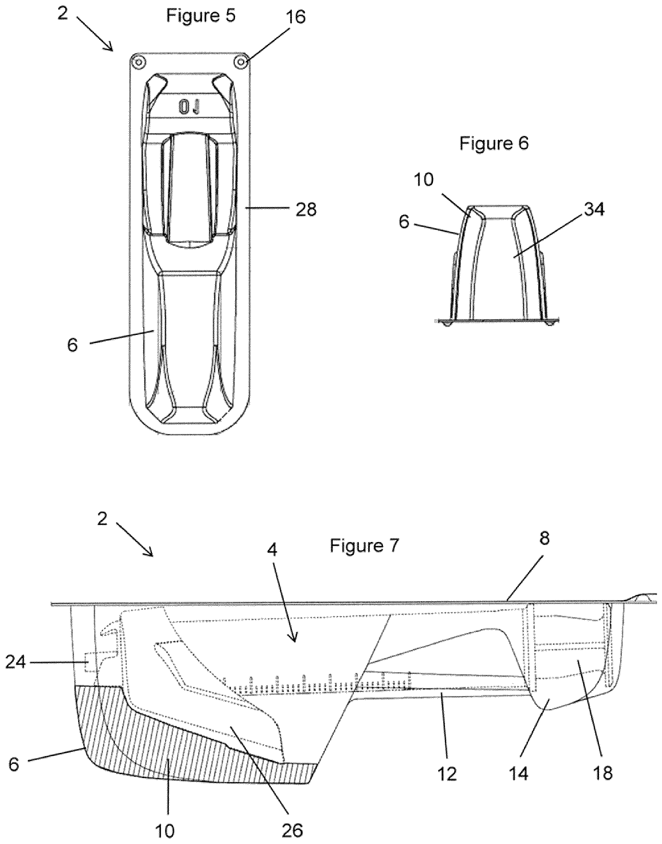

Figure 5 shows a view from the underside of the package 2, specifically from the base of the cavity 6. It can be seen that the peripheral portion 28 of the cavity 6 extends around the whole outer perimeter of the cavity 6. This is to ensure that when the backing 8 is sealed to the cavity 6 that the syringe 4 is completely sealed within the package 2. Also visible in this Figure are the protrusions 16. There is a protrusion 16 located at both corners at one end of the package 2, in this embodiment the opposite end to the fluid transfer tip 24. The purpose of the two protrusions 16 is to make it easier for the user, whether they are left or right handed, to remove the backing 8.

Figure 6 shows an end-on view of the package 2 and it can be seen that the end portion of the front pocket end 10 narrows to a thinner portion 34. The purpose of this is to follow the shape of the syringe 4, particularly the fluid transfer tip 24, in order to restrict general movement of the syringe 4 within the package 2.

The front pocket 10 within the cavity 6 is illustrated by the dashed region on Figure 7. The front pocket 10 provides a space for the fluid transfer tip 24 and lever member 26 of the syringe to pivot into once the backing 8 has been removed. The depth of the front pocket 10 is such that a portion of the plunger 18 protrudes out of the cavity 6 which is appropriate for a user to grab. In the embodiment shown, the shape of the front pocket 10 is shaped to accommodate features of the syringe, for example the lever member 26. It is also possible to see in this Figure that, when the backing 8 is still sealed to the cavity 6, the plunger 18 of the syringe 4 rests against the backing 8 and this prevents it from tipping. Therefore, as soon as the backing 8 is removed, the syringe 4 is free to pivot. The backing 8 is made from a sufficiently strong material that this pressure does not weaken the backing 8 to the point where the plunger 18 may break through it. This is particularly relevant as during transportation the device may move within the packaging 2, which may result in the plunger 18 repeatedly pressing against the backing 8, potentially weakening the material.

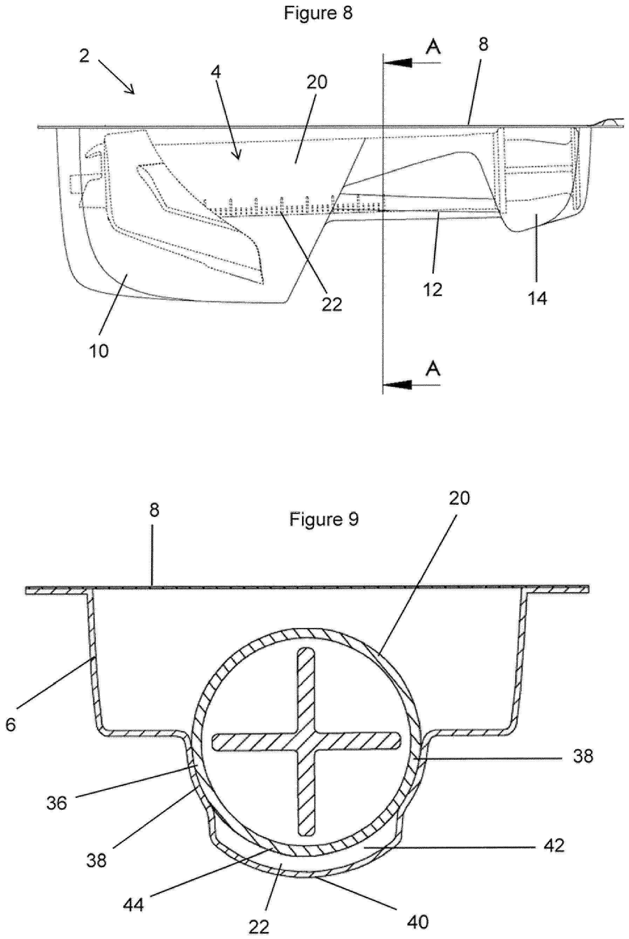

Another issue associated with the packaging of medical devices, such as the syringe 4 contained in a blister-type packaging 2, is that both high and low frequency vibrations which arise during storage and transportation can cause markings 22 on the device to wear away. Figure 8 shows how the embodiment of the syringe packaging 2 helps to reduce these effects. On the central portion 12 of the cavity 6, where the syringe barrel 20 comes into contact with a wall of the cavity 6, there is provided a recess such that the cavity 6 only comes into contact with opposed side portions of the barrel 20, rather than a lower portion where the scale 22 is typically marked. Accordingly the supporting surface walls of the cavity 6 are located away from the printed markings 22 on the syringe 4.

Figure 9 shows a cross-sectional view through the line A-A of Fig. 8. It can be seen that the syringe barrel 20 is supported on the side portions 36 of its circumference by the cavity side walls 38. The lower portion 40 of the cavity 6 is shaped such that there is a space 42 between the lower portion 40 of the cavity 6 and the lower portion 44 of the barrel 20, such that the lower portion 44 of the barrel 20 does not come into contact with the cavity and therefore the amount of wearing of the markings 22 is reduced.

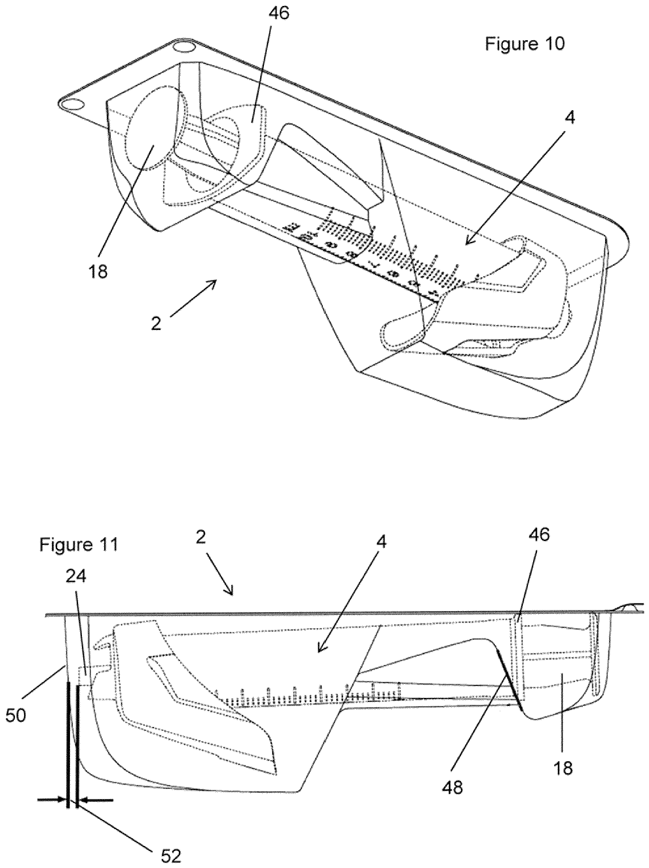

As seen in Figure 10 the width of the cavity 6, more specifically the front pocket 10, is similar to that of the syringe 4. In the embodiment shown, where the syringe 4 also comprises a lever member 24, the syringe 4 is prevented from rotating axially within the cavity 6 as the lever member 24 would quickly come into contact with the side walls of the cavity 6 and be prevented from further rotating. This ensures that the syringe 4 remains in this orientation such that it does not come into contact with the walls of the cavity 6. The finger grip portion 46 proximal to the plunger 18 also assists in preventing the syringe from rotating. The syringe is supported on the sides of the syringe rather than being supported from the bottom. Fig. 10 also shows a syringe 4 inside the package 2 and it can be seen that the syringe barrel 20 has a finger grip portion 46. This finger grip portion 46 positioned in the rear pocket 14 can also act to prevent the syringe 4 from rotating, this ensures that the markings on the barrel 20 remain in the position shown in Fig. 9.

Figure 11 shows the finger grip portion 46 of the syringe 4 abutting against an inside wall 48 of the rear pocket 14. As the finger grip portion 46 abuts against this wall 48 it prevents the syringe 4 from sliding forward in the package 2 and thus prevents the fluid transfer tip 24 from coming into contact with the end wall 50 of the cavity 6. It keeps a space 52 shown by the lines in the Figure. The wall 48 helps to ensure that the fluid transfer tip does not come into contact with the cavity wall 50 even as the syringe 4 pivots within the cavity.

Figure 12 illustrates the various stages of opening the packaging 2. As shown in Fig. 12a, a user is able to hold the packaging 2 by the cavity 6 in one hand 54 and peel the backing off using a second hand 56. The user peels the backing 8 by pulling on a tab at the end proximal to the plunger 18 of the syringe 4 within the packaging 2. Fig. 12b illustrates how peeling the backing 8 away from the cavity 6 allows the syringe 4 to pivot within the cavity 6 such that the plunger 18 of the syringe 4 presents itself out of the cavity 6. It can also be seen in this Figure how the fluid transfer tip 24 moves down into the front pocket 10 of the cavity. Fig. 12c illustrates how, when the backing 8 is peeled away by a sufficient amount, the tip 24 moves fully into the front pocket 10 and the plunger 18 of the syringe 4 protrudes sufficiently such that it can easily be grabbed by a user.

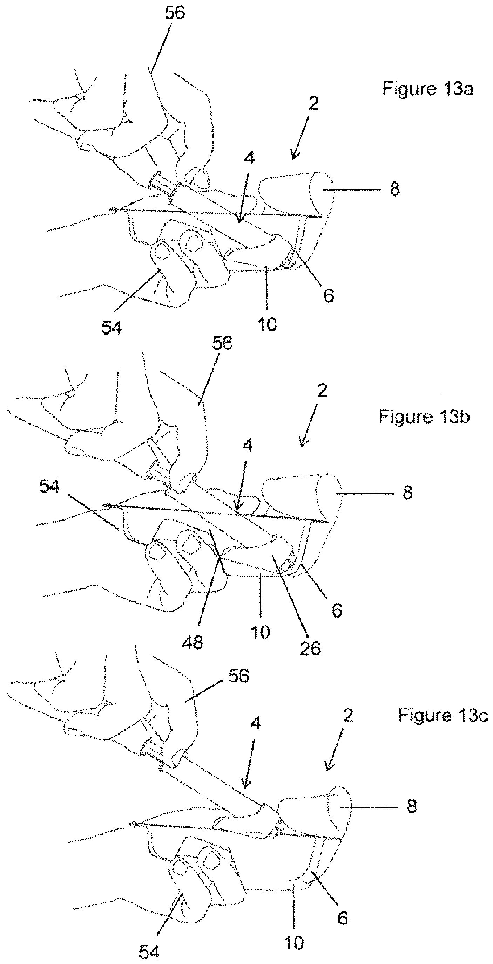

Figure 13 illustrates the various stages of the removal of the syringe 4 from the packaging 2 by pulling the syringe 4 out by hand. Fig. 13a illustrates how a user grabs the plunger end of the syringe 4 which is protruding out of the cavity 6 and then proceeds to pull with both a horizontal and vertical component. It can be seen in this Figure that the backing 8 is peeled completely away from the cavity 6, however this may not be necessary and it may be possible for the syringe 4 to be removed without completely removing the backing 8. Fig. 13b shows how, when the syringe 4 is pulled out of the packaging 3, the lever member 26 slides along an inside wall 48 of the cavity 6. The angle of this wall 48 is such that it facilitates sliding of the lever member 26 out of the packaging 2. Fig. 13c shows the syringe 4 removed completely from the packaging 2 without the user coming into contact with the fluid transfer tip 24 of the syringe 4.

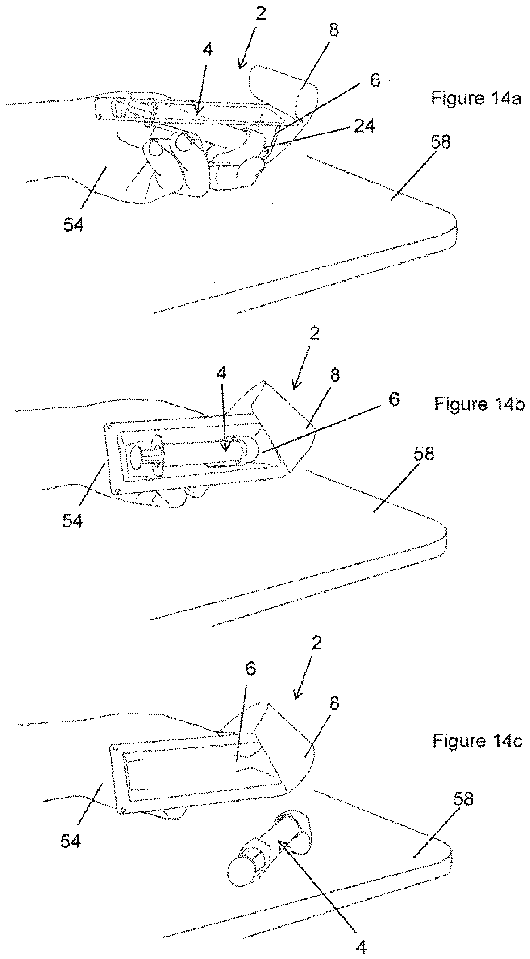

Figure 14 illustrates the various stages of the removal of the syringe 4 from the packaging 2 in an aseptic situation. As shown in Fig. 14a, a user is able to firstly remove the backing 8 from the cavity 6 by peeling it from the tab located proximal to the plunger end of the syringe 4. This minimises the chances of the user coming into contact with the fluid transfer tip 24 of the syringe 4 thus avoiding contamination. Once the backing 8 is removed the user is then able to hold the cavity 6 in one hand 54 as seen in Fig. 14b. The user is then able to tilt the packaging 2 such that the syringe 4 begins to tip out of the cavity 6. The package is fully tilted as seen in Fig. 14c and the syringe 4 then falls out of the cavity 6, this can be onto a disinfected Main General Aseptic Field 58 such as a portable tray or a procedure trolley for surgical use, or a Main Critical Aseptic Field such as a sterilised drape.

In the embodiments shown the cavity 6 is formed from a single piece of plastic with an internal structure which provides the fulcrum 32. However it will be appreciated that this may be achieved by alternative means, for example a less structured blister package could be used and an insert could be placed into the cavity of the blister package to provide the fulcrum.

Although it has been identified that it is advantageous for the plunger end of the device to protrude from the cavity when the backing is removed, it will be appreciated that in certain situations it may be advantageous for the fluid transfer tip to protrude from the cavity. In such situations the pivot point could be arranged so that this would tend to happen. It will also be appreciated that the syringe or other medical device may, additionally, be able to adopt one or more positions in the package where it does not tend to pivot. For example, the device may be able to slide within the cavity of the package. In such a scenario a user would be able to first peel away the backing to open the cavity and then tilt the package slightly such that the centre of mass of the device moves forward of the fulcrum, thus causing the device to pivot and present one end.

Although in the embodiments shown the syringe 4 is removed from the package 2 whilst the package 2 is still being held in a user's hand, it is also envisaged that the backing 8 could be removed and the package 2 could be subsequently placed on a surface at which point the syringe 4 may be removed.

In the embodiments shown the backing 8 is sealed around a peripheral portion 28 of the top surface of the cavity 6 and when it is peeled away it is left in contact with one end of the cavity 6. However it will be appreciated that the backing may be completely removed from the device or may be attached or sealed by other suitable means. For example it may be designed such that only part of the backing is able to be peeled away to ensure that the fluid transfer tip remains in at least a partially sealed portion such that the user is only able to grab the plunger end of the syringe.

In the embodiments shown the syringe 4 comprises a lever member 26 which substantially affects the position of the centre of mass. It will be appreciated that the packaging can be used with other medical devices which do not comprise a lever member, in such cases the fulcrum is appropriately positioned such that the centre of mass can fall within the front pocket of the package thus allowing the medical device to pivot.

Figures 15 to 20 relate to alternative embodiments of a package containing a needle instead of a syringe, in particular a needle comprising a needle hub and a removable cover. There is seen in Figs. 15a and 15b a moulded plastic cavity 106 that can be closed by a removable backing (not shown) to form a sealed package. In a similar way to the embodiments described previously, the cavity 106 comprises a front pocket 110, a central portion 112 and a rear pocket 114. A fulcrum 132 is formed at a corner between the front pocket 110 and the central portion 112. In addition, the front pocket 110 is provided with a recess 111 at its forward end that can catch and retain a needle cover in use. This will be understood with reference to Figs. 16a-16i.

Fig. 16a shows an unopened package 102 comprising such a cavity 106 sealed by a removable backing 108. A needle hub 160 and needle cover 170 is contained inside the cavity 106. Fig. 16b shows the backing 108 being removed, the needle hub 160 tilting forwards into the front pocket 110, and a user connecting a syringe 180 to the needle hub 160, for example pushing a tapered fluid transfer tip into the needle hub to form a friction fit e.g. Luer slip-type connection. Fig. 16c shows a user pushing the syringe 180 and needle hub 160 forwards in the cavity 106 so that the needle cover 170 locks into the recess 111 at the front of the cavity 106. This is assisted by the angled shape of the front pocket 110.

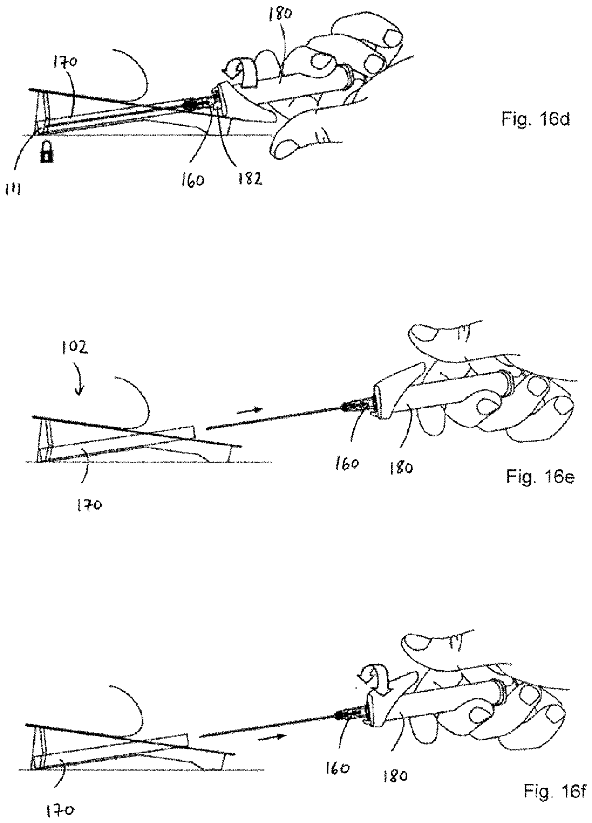

Fig. 16d shows an optional variant where the needle cover 170 has a square cross-section at its tip. The square shape of the recess 111 holds the needle cover 170 like a wrench and prevents it from rotating. Once the syringe 180 has been pushed forward to lock the needle cover 170 in the recess 111, a user can rotate the syringe 180 while the needle hub 160 is held stationary. This may allow a locking collar 182 to twist into engagement with the needle hub 160, for example providing a Luer lock-type connection in addition to a friction fit. Such connections are described in more detail in the Applicant's prior publications WO 2014/020090 and WO 2015/014914.

Fig. 16e shows a user withdrawing the syringe 180 with the needle hub 160 attached, leaving the needle cover 170 inside the package 102. Fig. 16f shows an optional variant where the needle cover 170 has a square cross-section at its tip and the syringe 180 is rotated while pulling to release the needle hub 160 from the cover 170.

After the syringe 180 has been used for a medical procedure, the needle hub 160 can be safely recapped with the needle cover 170 that is retained in the original package 102, as shown in Figs. 16g-16i. Fig. 16h shows how the syringe 180 may include a lever member 126 that is operated to disconnect the needle hub 160 once it is safely resheathed. The syringe 180 can then be withdrawn, with the needle hub 160 safely stored in the package 102, ready for disposal.

Figs. 17a-d show an alternative embodiment of the package 102, with an additional gripping part 190 inserted into the cavity to add additional gripping for the needle cover 170. This small piece of plastic or metal locks the needle cover 170 when the needle hub 160 is pushed forwards in the cavity, for example when connecting a syringe 180. The gripping part 190 may be seated in the recess 111 or take its place. Figs. 18a-d show another alternative embodiment of the package 102, with an additional gripping part 195 that can lock the needle cover 170 when the needle hub 160 is pushed forwards in the cavity, for example when connecting a syringe 180. An angled edge of the gripping part 195 allows for the needle cover 170 to enter through an aperture before it is locked permanently from removal.

There is seen in Fig. 19 a moulded plastic cavity 206 that can be closed by a removable backing (not shown) to form a sealed package. In a similar way to the embodiments described previously, the cavity 206 comprises a front pocket 210, a central portion 212 and a rear pocket 214. A fulcrum 232 is formed at a corner between the front pocket 210 and the central portion 212. In this embodiment, the front pocket 210 is provided with a recess 211 at its rear end that can catch and retain a needle cover in use. This will be understood with reference to Figs. 20a-20f.

Fig. 20a shows such a cavity 206 as part of an opened package 202 where the backing 208 has been removed. A syringe 280 connected to a needle hub 260 is pushed down into the recess 211 so that the needle cover 270 is caught. Fig. 20b shows the needle cover 270 held in the cavity 206 by friction. It is seen from Fig. 20c that the syringe 280 and needle hub 260 can be withdrawn, leaving the needle cover 270 with the package 202. After the syringe 280 has been used for a medical procedure, the needle hub 260 can be safely recapped with the needle cover 270 that is retained in the original package 202, as shown in Figs. 20d-20f. Fig. 20d shows the needle being placed back in the needle cover 270. Fig. 20e shows a user pressing a lever member 226 on the syringe 280 to release the needle hub 260. Fig. 20f shows the syringe 280 now detached from the needle hub 260, the needle left safely stored in the needle cover 270.

In Figs. 19 and 20 the cavity 206 comprises a front pocket 210 that is angled forwards and down from a fulcrum 232 so that a device contained in the cavity 206 tends to tip forwards and present itself for ease of removal once the backing 208 is removed. However, it is envisaged that such a package 202 may not always include this feature and the cavity 206 may simply provide a recess 211 to assist a user in removing a cover from the device - whether the device is a needle hub or other medical device.

Another embodiment of a package 302 is seen in Figure 21. The package 302 comprises a cavity 306 that can contain a medical device (not shown) and be sealed by a removable backing 308. The materials of the cavity 306 and backing 308 may be the same as previously described with respect to Figure 1. The package 302 is similar to the one described in relation to Figures 1-14 in terms of its general form and function. However the package 302 differs in that the cavity 306 comprises a front pocket 310, a rear pocket 314 and a central portion extending therebetween which is not flat but defines an additional central pocket 312. It can be seen that the central pocket 312 and rear pocket 314 both define an outer surface extending in the same plane, so that the package 302 can be supported stably on a horizontal surface with the front pocket 310 tilted to promote ease of removal of the device contained in the package 302.

The central pocket 312 may be used to contain another component separately from the medical device, for example a cap for the device. As soon as the package is opened, a device contained in the cavity 306 will pivot forward into the front pocket 310, as previously described, so as to present its rear end to a user. If the device is not to be used straightaway for a medical procedure, then a user can grasp the device at its rear end to remove it from the cavity 306 and then push the front end of the device down into engagement with the cap (or other component) contained in the central pocket 312. The central pocket 312 may be shaped to grip the cap, for example so that the device can be twisted where the cap requires a screw connection. The front end of the device, which may for example comprise the fluid transfer tip of a syringe, does not come into contact with the user or the external environment and can be kept sterile while covered by the cap. The package 302 therefore provides a complete solution ensuring that the aseptic technique is not compromised.

While different variants of a package and cavity have been described separately above, it will be understood that a single package could combine one or more such cavities, for example with a shared backing. For instance, a package could include one cavity for a needle hub and another cavity for a syringe, or one cavity for a catheter and another for a catheter connector.

When sealing a package that contains a medical device such as a syringe, a potential problem is that the device may tend to pivot within the package such that its rear end e.g. the plunger of a syringe protrudes from the cavity. The protruding plunger can interfere when applying the backing which seals the package. This can be overcome by including a step within the sealing process which supports the device in an unstable e.g. horizontal position such that it does not pivot forward with its rear end protruding from the cavity. This can be achieved in many ways, but in the following Figures and discussion a U-shaped clip is described which supports, e.g. lifts and holds, a syringe in a horizontal position in a package.

Figure 22 shows a U-shaped clip 60 which can be used in conjunction with any of the packages 2, 102, 202, 302 shown in previous Figures. The U-shaped clip 60 is formed of a relatively rigid material with a flat base 62 and side walls comprised of a lower vertical portion 64 and a top flared portion 66.

Figure 23 shows the U-shaped clip 60 applied to the package 2. The U-shaped clip 60 can be pressed onto the outside of the cavity 6 proximal to the front pocket 10. This Figure shows how the U-shaped clip 60 can be pushed into place until the base 62 of the U-shaped clip 60 is flush with the bottom surface 68 of the front pocket 10. The width of the lower portion of the U-shaped clip 60 defined by the lower vertical portions 64 is narrower than the front pocket 10. This causes the walls of the cavity 6 in the front pocket 10 to deform when the U-shaped clip 60 is pushed into place. This forces the syringe 4 within the cavity 6 to pivot into a horizontal position and be supported in this position allowing the backing 8 to be applied without interference.

Figure 24 shows a side view of the package 2 before being placed onto the U-shaped clip 60. It can be seen that the syringe 4 is naturally in a pivoted position within the package 2 such that lever member 26 and fluid transfer tip 24 are pivoted forward into the front pocket 10. In the pivoted position the plunger 18, at the rear end of the syringe 4, protrudes from the cavity 6. The protruding plunger 18 can cause difficulties when attaching the backing 8 to the cavity 6 during manufacture of the package 2. With the plunger 18 protruding from the cavity 6 it would be necessary to push the plunger 18 down as the backing 8 (seen in Fig. 23) is attached and sealed. This may cause the backing 8 to be loose. This is particularly undesirable as the syringe 4 would be free to move around within the package 2. By pressing the package 2 into the U-shaped clip 60 this ensures that the syringe 4 is horizontal within the cavity 6 thus the backing 8 can be applied without any obstructions. Also shown in this Figure is an optional further attachment 70. The attachment 70 acts to support the rear pocket 14 of the cavity 6. The attachment 70 may be integrally moulded as part of the clip 60 and attached by an elongate member 72. The attachment 70 acts to keep the cavity 6 horizontal which may further assist during manufacture when applying the backing 8.

Figure 25 shows a cross-sectional view through the line A-A in Figure 24. When the U-shaped clip 60 is not attached to the cavity 6 it can be seen that the syringe 4 is pivoted forward within the cavity 6 such that the lever member 26 sits in the bottom of the front pocket 10. The flared portions 66 of the U-shaped clip 60 assist in guiding the cavity 6 into the U-shaped clip 60 in the direction shown by an arrow.

Figure 26 shows the U-shaped clip 60 attached to the cavity 6. The vertical portions 64 of the clip 60 act to squeeze together the walls of the front pocket 10 and deform the cavity 6 so that the syringe 4 cannot pivot forward into a stable position. It can be seen in this Figure that the syringe 4 is supported in a horizontal position within the cavity 6 such that the plunger 18 is contained within the rear pocket 14. In this position the backing 8 can easily be attached to the cavity 6.

Figure 27 shows a cross sectional view through the line B-B in Figure 26. The side walls of the cavity 6, specifically to the walls of the front pocket 10, have been deformed by the U-shaped clip 60. As the U-shaped clip 60 is pushed into place, the force applied by its vertical portions 64 deforms the side walls of the front pocket 10 and subsequently forces the lever member 26 which is attached to the syringe 4 upwards to be supported in a horizontal position.

The flared shape of the U-shaped clip 60 is particularly advantageous as the motion of the cavity 6 being pressed onto the U-shaped clip 60 assists in pushing the syringe 4 upwards. If for instance the side walls of the cavity 6 were just pressed inwards, this may not necessarily cause the syringe 4 to pivot upward and instead it may just hold it in place in a stable position.

Although it is shown that the syringe 4 is contained within the cavity 6 and then the U-shaped clip 60 is attached it may be possible for the cavity 6 to be deformed by applying the clip 60 before the syringe 4 is placed into the cavity 6. This would mean that the syringe 4 is inserted into the cavity 6 and is instantly supported in a horizontal position.

Although the U-shaped clip 60 is shown to be positioned on the front pocket 10, it is envisaged that it could be positioned anywhere on the cavity 6 such that it causes deformation resulting in the syringe 4 being supported in an unstable horizontal position within the cavity 6.

It will be appreciated that the U-shaped clip 60 may form part of a machine in the manufacturing process and depending on the type of machine there may also be an array of such U-shaped clips 60.

Although the U-shaped clip 60 is discussed with reference to a manufacturing and sealing process for the package 2, it is envisaged that it could be utilised for other purposes. For example the U-shaped clip 60 could remain attached to the front pocket 10 of the cavity 6 until the user wishes to release the syringe 4. In such an example the user may peel away the backing 8 and the U-shaped clip 60 may hold the syringe in place until the user wishes to remove the syringe 4. When the user wishes to remove the syringe 4 he may then simply remove the U-shaped clip 60 so that the syringe 4 is free to pivot forward into a more stable position.

## Claims

1. A sealed package (2; 102; 202; 302) containing a medical device (4; 160, 170; 260, 270) that has a predefined centre of mass, the package (2; 102; 202; 302) comprising:
a cavity (6; 106; 206; 306) containing the device (4; 160, 170; 260, 270);
a removable backing (8; 108; 208; 308) that seals the device (4; 160, 170; 260, 270) inside the cavity (6; 106; 206; 306); **characterised in that** the sealed package further comprises:
a fulcrum (32; 132; 232) within the cavity (6; 106; 206; 306), defined as a point of contact between the device (4; 160, 170; 260, 270) and a wall of the cavity (6; 106; 206; 306), the fulcrum (32; 132; 232) being positioned such that the predefined centre of mass of the device (4; 160, 170; 260, 270) is in a forward direction relative to the fulcrum (32; 132; 232) so that the device (4; 160, 170; 260, 270) tends to pivot about the fulcrum (32; 132; 232) into a stable position; and
a front pocket (10; 110; 210; 310) formed in the cavity (6; 106; 206; 306) which extends in said forward direction and below the fulcrum (32; 132; 232) to receive a front end of the device (4; 160, 170; 260, 270) when the device (4; 160, 170; 260, 270) is free to pivot into a stable position.

2. A sealed package (2; 102; 202; 302) according to claim 1, wherein the device (4; 160, 170; 260, 270) is contained in the cavity (6; 106; 206; 306) in an unstable position.

3. A sealed package (2; 102; 202; 302) according to claim 1 or 2, wherein the removable backing (8; 108; 208; 308) is arranged to substantially prevent the device (4; 160, 170; 260, 270) from pivoting forward into a stable position.

4. A sealed package (2; 102; 202; 302) according to any preceding claim, wherein the device (4; 160, 170; 260, 270) is contained in an unstable position with the removable backing (8; 108; 208; 308) forming a surface of the sealed package (2; 102; 202; 302) that is in contact with a rear end (18; 160; 260) of the device (4; 160, 170; 260, 270) and wherein optionally the cavity (6; 106; 206; 306) is arranged such that, when the backing (8; 108; 208; 308) is removed, the device (4; 160, 170; 260, 270) is free to pivot forward so that the rear end (18; 160; 260) of the device (4; 160, 170; 260, 270) projects out of the cavity (6; 106; 206; 306) beyond said surface.

5. A sealed package (2; 102; 202; 302) according to claim 4, wherein the front pocket (10; 110; 210; 310) extends below the fulcrum (32; 132; 232) to such a depth that the rear end (18; 160; 260) of the device (4; 160, 170; 260, 270) projects out of the cavity (6; 106; 206; 306) beyond said surface when the forward end is received in the front pocket (10; 110; 210; 310).

6. A sealed package (102) according to any preceding claim, wherein the front pocket (110) comprises a catch (111) arranged to hold the front end once the device (160, 170) has pivoted forward into a stable position.

7. A sealed package (102; 202; 302) according to any preceding claim, wherein the front end comprises a removable cover (170; 270) and wherein optionally the cavity (106; 206; 306) comprises means (111; 190; 195; 211; 312) for gripping the removable cover (170; 270).

8. A sealed package (2; 102; 202; 302) according to any preceding claim, wherein the cavity (6; 106; 206; 306) comprises a central portion (12; 112) having a central depth and the front pocket (10; 110; 210; 310) is defined by a forward portion of the cavity (6; 106; 206; 306) having a depth that is greater and wherein optionally the fulcrum (32; 132; 232) is formed by a corner in a wall of the cavity (6; 106; 206; 306) between the front pocket (10; 110; 210; 310) and the central portion (12; 112).

9. A sealed package (302) according to claim 8, wherein the central portion comprises a central pocket (312) having a depth that is less than the depth of the front pocket (310) and optionally further comprising an additional component for use with the medical device that is contained in the central pocket (312).

10. A sealed package (2) according to claim 8 or 9, wherein the central portion (12) comprises side walls (38) that support the device (4) and a lower wall (40) that is spaced away from the device (2).

11. A sealed package (2; 102; 202; 302) according to any preceding claim, wherein the cavity (6; 106; 206; 306) has an outer profile below the device (4; 160, 170; 260, 270) which is shaped such that, when the package (2; 102; 202; 302) rests on a horizontal surface, the predefined centre of mass of the device (4; 160, 170; 260, 270) is forward of the fulcrum (32; 132; 232).

12. A sealed package (2; 102; 202; 302) according to any preceding claim, wherein the medical device comprises a fluid transfer device (4) or wherein the medical device consists of a needle hub (160; 260), carrying a needle, and a needle cover (170; 270).

13. A sealed package (2; 102; 202; 302) according to any preceding claim, further comprising a support (60) arranged outside the package (2; 102; 202; 302) to deform the cavity (6; 106; 206; 306) so as to substantially prevent the device (4; 160, 170; 260, 270)) from pivoting forward into a stable position inside the package (2; 102; 202; 302).

14. A method of sealing a package (2; 102; 202; 302) containing a medical device (4; 160, 170; 260, 270) that has a predefined centre of mass, the package (2; 102; 202; 302) comprising:
a cavity (6; 106; 206; 306) containing the medical device (4; 160, 170; 260, 270);
a fulcrum (32; 132; 232) within the cavity, defined as a point of contact between the device (4; 160, 170; 260, 270) and a wall of the cavity (6; 106; 206; 306), the fulcrum (32; 132; 232) being positioned such that the predefined centre of mass of the device (4; 160, 170; 260, 270) is in a forward direction relative to the fulcrum (32; 132; 232) so that the device (4; 160, 170; 260, 270) tends to pivot about the fulcrum (32; 132; 232) into a stable position; and
a front pocket (10; 110; 210; 310) formed in the cavity (6; 106; 206; 306) which extends in said forward direction and below the fulcrum (32; 132; 232) to receive a front end of the device (4; 160, 170; 260, 270) when the device (4; 160, 170; 260, 270) is free to pivot into a stable position,
the method comprising:
deforming one or more walls of the cavity (6; 106; 206; 306) such that the medical device (4; 160, 170; 260, 270) contained in the cavity (6; 106; 206; 306) is supported on the fulcrum (32; 132; 232) in an unstable position inside the package (2; 102; 202; 302); and
applying a removable backing (8; 108; 208; 308) to seal the cavity (6; 106; 206; 306).

15. A method according to claim 14, comprising deforming one or more walls of the front pocket (10; 110; 210; 310).

## Patentansprüche

1. Versiegelte Verpackung (2; 102; 202; 302) beinhaltend eine medizinische Vorrichtung (4; 160, 170; 260, 270), die einen vordefinierten Masseschwerpunkt hat, wobei die Verpackung (2; 102; 202; 302) umfasst:
einen Hohlraum (6; 106; 206; 306), der die Vorrichtung (4; 160, 170; 260, 270) beinhaltet;
eine abnehmbare Rückseite (8; 108; 208; 308), die die Vorrichtung (4; 160, 170; 260, 270) im Inneren des Hohlraums (6; 106; 206; 306) versiegelt, **dadurch gekennzeichnet, dass** die versiegelte Verpackung weiter umfasst:
einen Schwenkpunkt (32; 132; 232) im Inneren des Hohlraums (6; 106; 206; 306), der als Kontaktpunkt zwischen der Vorrichtung (4; 160, 170; 260, 270) und einer Wand des Hohlraums (6; 106; 206; 306) definiert ist, wobei der Schwenkpunkt (32; 132; 232) so positioniert ist, dass der vordefinierte Masseschwerpunkt der Vorrichtung (4; 160, 170; 260, 270) in einer Vorwärtsrichtung relativ zum Schwenkpunkt (32; 132; 232) liegt, sodass die Vorrichtung (4; 160, 170; 260, 270) dazu neigt, um den Schwenkpunkt (32; 132; 232) herum in eine stabile Position zu schwenken; und
eine Vordertasche (10; 110; 210; 310), die im Hohlraum (6; 106; 206; 306) gebildet ist, die sich in die Vorwärtsrichtung und unter dem Schwenkpunkt (32; 132; 232) erstreckt, um ein vorderes Ende der Vorrichtung (4; 160, 170; 260, 270) aufzunehmen, wenn die Vorrichtung (4; 160, 170; 260, 270) frei in eine stabile Position schwenken kann.

2. Versiegelte Verpackung (2; 102; 202; 302) nach Anspruch 1, wobei die Vorrichtung (4; 160, 170; 260, 270) im Hohlraum (6; 106; 206; 306) in einer instabilen Position beinhaltet ist.

3. Versiegelte Verpackung (2; 102; 202; 302) nach Anspruch 1 oder 2, wobei die abnehmbare Rückseite (8; 108; 208; 308) angeordnet ist, um im Wesentlichen zu verhindern, dass die Vorrichtung (4; 160, 170; 260, 270) nach vorne in eine stabile Position schwenkt.

4. Versiegelte Verpackung (2; 102; 202; 302) nach einem der vorstehenden Ansprüche, wobei die Vorrichtung (4; 160, 170; 260, 270) in einer instabilen Position beinhaltet ist, wobei die abnehmbare Rückseite (8; 108; 208; 308) eine Oberfläche der versiegelten Verpackung (2; 102; 202; 302) bildet, die mit einem hinteren Ende (18; 160; 260) der Vorrichtung (4; 160, 170; 260, 270) in Kontakt ist, und wobei optional der Hohlraum (6; 106; 206; 306) so angeordnet ist, dass, wenn die Rückseite (8; 108; 208; 308) abgenommen wird, die Vorrichtung (4; 160, 170; 260, 270) frei nach vorne schwenken kann, sodass das hintere Ende (18; 160; 260) der Vorrichtung (4; 160, 170; 260, 270) aus dem Hohlraum (6; 106; 206; 306) über die Oberfläche hinausragt.

5. Versiegelte Verpackung (2; 102; 202; 302) nach Anspruch 4, wobei sich die Vordertasche (10; 110; 210; 310) unter dem Schwenkpunkt (32; 132; 232) bis zu einer solchen Tiefe erstreckt, dass das hintere Ende (18; 160; 260) der Vorrichtung (4; 160, 170; 260, 270) aus dem Hohlraum (6; 106; 206; 306) über die Oberfläche hinausragt, wenn das vordere Ende in der Vordertasche (10; 110; 210; 310) aufgenommen ist.

6. Versiegelte Verpackung (102) nach einem der vorstehenden Ansprüche, wobei die Vordertasche (110) einen Haken (111) umfasst, der angeordnet ist, das vordere Ende zu halten, sobald die Vorrichtung (160, 170) nach vorne in eine stabile Position geschwenkt ist.

7. Versiegelte Verpackung (102; 202; 302) nach einem der vorstehenden Ansprüche, wobei das vordere Ende eine abnehmbare Abdeckung (170; 270) umfasst und wobei der Hohlraum (106; 206; 306) optional Mittel (111; 190; 195; 211; 312) zum Ergreifen der abnehmbaren Abdeckung (170; 270) umfasst.

8. Versiegelte Verpackung (2; 102; 202; 302) nach einem der vorstehenden Ansprüche, wobei der Hohlraum (6; 106; 206; 306) einen zentralen Abschnitt (12; 112) mit einer zentralen Tiefe umfasst und die Vordertasche (10; 110; 210; 310) durch einen vorderen Abschnitt des Hohlraums (6; 106; 206; 306) mit einer größeren Tiefe definiert ist, und wobei der Schwenkpunkt (32; 132; 232) optional durch eine Ecke in einer Wand des Hohlraums (6; 106; 206; 306) zwischen der Vordertasche (10; 110; 210; 310) und dem zentralen Abschnitt (12; 212) gebildet ist.

9. Versiegelte Verpackung (302) nach Anspruch 8, wobei der zentrale Abschnitt eine zentrale Tasche (312) mit einer Tiefe umfasst, die geringer als die Tiefe der Vordertasche (310) ist, und optional weiter eine zusätzliche Komponente zur Verwendung mit der medizinischen Vorrichtung umfasst, die in der zentralen Tasche (312) beinhaltet ist.

10. Versiegelte Verpackung (2) nach Anspruch 8 oder 9, wobei der zentrale Abschnitt (12) Seitenwände (38), die die Vorrichtung (4) stützen, und eine untere Wand (40), die von der Vorrichtung (2) beabstandet ist, umfasst.

11. Versiegelte Verpackung (2; 102; 202; 302) nach einem der vorstehenden Ansprüche, wobei der Hohlraum (6; 106; 206; 306) ein Außenprofil unter der Vorrichtung (4; 160, 170; 260, 270) hat, das so geformt ist, dass, wenn die Verpackung (2; 102; 202; 302) auf einer horizontalen Oberfläche liegt, der vordefinierte Masseschwerpunkt der Vorrichtung (4; 160, 170; 260, 270) vorderhalb des Schwenkpunktes (32; 132; 232) liegt.

12. Versiegelte Verpackung (2; 102; 202; 302) nach einem der vorstehenden Ansprüche, wobei die medizinische Vorrichtung eine Fluidübertragungsvorrichtung (4) umfasst oder wobei die medizinische Vorrichtung aus einem Nadelansatzstück (160; 260), das eine Nadel trägt, und einer Nadelabdeckung (170; 270) besteht.

13. Versiegelte Verpackung (2; 102; 202; 302) nach einem der vorstehenden Ansprüche, weiter umfassend einen Träger (60), der außerhalb der Verpackung (2; 102; 202; 302) angeordnet ist, um den Hohlraum (6; 106; 206; 306) zu verformen, um im Wesentlichen zu verhindern, dass die Vorrichtung (4; 160, 170; 260, 270) nach vorne in eine stabile Position im Inneren der Verpackung (2; 102; 202; 302) schwenkt.

14. Verfahren zum Versiegeln einer Verpackung (2; 102; 202; 302) beinhaltend eine medizinische Vorrichtung (4; 160, 170; 260, 270), die einen vordefinierten Masseschwerpunkt hat, wobei die Verpackung (2; 102; 202; 302) umfasst:
einen Hohlraum (6; 106; 206; 306), der die Vorrichtung (4; 160, 170; 260, 270) beinhaltet;
einen Schwenkpunkt (32; 132; 232) im Inneren des Hohlraums, der als Kontaktpunkt zwischen der Vorrichtung (4; 160, 170; 260, 270) und einer Wand des Hohlraums (6; 106; 206; 306) definiert ist, wobei der Schwenkpunkt (32; 132; 232) so positioniert ist, dass der vordefinierte Masseschwerpunkt der Vorrichtung (4; 160, 170; 260, 270) in einer Vorwärtsrichtung relativ zum Schwenkpunkt (32; 132; 232) liegt, sodass die Vorrichtung (4; 160, 170; 260, 270) dazu neigt, um den Schwenkpunkt (32; 132; 232) herum in eine stabile Position zu schwenken; und
eine Vordertasche (10; 110; 210; 310), die im Hohlraum (6; 106; 206; 306) gebildet ist, die sich in die Vorwärtsrichtung und unter dem Schwenkpunkt (32; 132; 232) erstreckt, um ein vorderes Ende der Vorrichtung (4; 160, 170; 260, 270) aufzunehmen, wenn die Vorrichtung (4; 160, 170; 260, 270) frei in eine stabile Position schwenken kann,
das Verfahren umfassend:
Verformen einer Wand oder mehrerer Wände des Hohlraums (6; 106; 206; 306), sodass die medizinische Vorrichtung (4; 160, 170; 260, 270), die im Hohlraum (6; 106; 206; 306) beinhaltet ist, auf dem Schwenkpunkt (32; 132; 232) in einer instabilen Position im Inneren der Verpackung (2; 102; 202; 302) gestützt ist; und
Aufbringen einer abnehmbaren Rückseite (8; 108; 208; 308), um den Hohlraum (6; 106; 206; 306) zu versiegeln.

15. Verfahren nach Anspruch 14, umfassend ein Verformen einer Wand oder mehrerer Wände der Vordertasche (10; 110; 210; 310).

## Revendications

1. Emballage scellé (2 ; 102 ; 202 ; 302) contenant un dispositif médical (4 ; 160, 170 ; 260, 270) qui a un centre de gravité prédéfini, l'emballage (2 ; 102 ; 202 ; 302) comprenant :
une cavité (6 ; 106 ; 206 ; 306) contenant le dispositif (4 ; 160, 170 ; 260, 270) ;
un dos amovible (8 ; 108 ; 208 ; 308) qui scelle le dispositif (4 ; 160, 170 ; 260, 270) à l'intérieur de la cavité (6 ; 106 ; 206 ; 306) ; **caractérisé en ce que** l'emballage scellé comprend en outre :
un point d'appui (32 ; 132 ; 232) dans la cavité (6 ; 106 ; 206 ; 306), défini comme un point de contact entre le dispositif (4 ; 160, 170 ; 260, 270) et une paroi de la cavité (6 ; 106 ; 206 ; 306), le point d'appui (32 ; 132 ; 232) étant positionné de telle sorte que le centre de gravité prédéfini du dispositif (4 ; 160, 170 ; 260, 270) est dans une direction vers l'avant par rapport au point d'appui (32 ; 132 ; 232) de telle manière que le dispositif (4 ; 160, 170 ; 260, 270) a tendance à pivoter autour du point d'appui (32 ; 132 ; 232) jusque dans une position stable ; et
une poche avant (10 ; 110 ; 210 ; 310) formée dans la cavité (6 ; 106 ; 206 ; 306) qui s'étend dans ladite direction vers l'avant et au-dessous du point d'appui (32 ; 132 ; 232) pour recevoir une extrémité avant du dispositif (4 ; 160, 170 ; 260, 270) lorsque le dispositif (4 ; 160, 170 ; 260, 270) est libre de pivoter jusque dans une position stable.

2. Emballage scellé (2 ; 102 ; 202 ; 302) selon la revendication 1, dans lequel le dispositif (4 ; 160, 170 ; 260, 270) est contenu dans la cavité (6 ; 106 ; 206 ; 306) dans une position instable.

3. Emballage scellé (2 ; 102 ; 202 ; 302) selon la revendication 1 ou 2, dans lequel le dos amovible (8 ; 108 ; 208 ; 308) est agencé pour empêcher sensiblement le dispositif (4 ; 160, 170 ; 260, 270) de pivoter vers l'avant jusque dans une position stable.

4. Emballage scellé (2 ; 102 ; 202 ; 302) selon l'une quelconque des revendications précédentes, dans lequel le dispositif (4 ; 160, 170 ; 260, 270) est contenu dans une position instable avec le dos amovible (8 ; 108 ; 208 ; 308) formant une surface de l'emballage scellé (2 ; 102 ; 202 ; 302) qui est en contact avec une extrémité arrière (18 ; 160 ; 260) du dispositif (4 ; 160, 170 ; 260, 270) et dans lequel facultativement la cavité (6 ; 106 ; 206 ; 306) est agencée de telle sorte que, lorsque le dos (8 ; 108 ; 208 ; 308) est retiré, le dispositif (4 ; 160, 170 ; 260, 270) est libre de pivoter vers l'avant de sorte que l'extrémité arrière (18 ; 160 ; 260) du dispositif (4 ; 160, 170 ; 260, 270) fait saillie hors de la cavité (6 ; 106 ; 206 ; 306) au-delà de ladite surface.

5. Emballage scellé (2 ; 102 ; 202 ; 302) selon la revendication 4, dans lequel la poche avant (10 ; 110 ; 210 ; 310) s'étend sous le point d'appui (32 ; 132 ; 232) jusqu'à une profondeur telle que l'extrémité arrière (18 ; 160 ; 260) du dispositif (4 ; 160, 170 ; 260, 270) fait saillie hors de la cavité (6 ; 106 ; 206 ; 306) au-delà de ladite surface lorsque l'extrémité avant est reçue dans la poche avant (10 ; 110 ; 210 ; 310).

6. Emballage étanche (102) selon l'une quelconque des revendications précédentes, dans lequel la poche avant (110) comprend une attache (111) agencée pour maintenir l'extrémité avant une fois que le dispositif (160, 170) a pivoté vers l'avant jusque dans une position stable.

7. Emballage scellé (102 ; 202 ; 302) selon l'une quelconque des revendications précédentes, dans lequel l'extrémité avant comprend un couvercle amovible (170; 270) et dans lequel facultativement la cavité (106; 206; 306) comprend des moyens (111; 190 ; 195 ; 211 ; 312) pour saisir le couvercle amovible (170 ; 270).

8. Emballage scellé (2 ; 102 ; 202 ; 302) selon l'une quelconque des revendications précédentes, dans lequel la cavité (6 ; 106 ; 206 ; 306) comprend une partie centrale (12 ; 112) ayant une profondeur centrale et la poche avant (10 ; 110 ; 210 ; 310) est définie par une partie avant de la cavité (6 ; 106 ; 206 ; 306) ayant une profondeur qui est supérieure et dans lequel facultativement le point d'appui (32 ; 132 ; 232) est formé par un coin dans une paroi de la cavité (6 ; 106 ; 206 ; 306) entre la poche avant (10 ; 110 ; 210 ; 310) et la partie centrale (12 ; 112).

9. Emballage scellé (302) selon la revendication 8, dans lequel la partie centrale comprend une poche centrale (312) ayant une profondeur qui est inférieure à la profondeur de la poche avant (310) et comprenant en outre facultativement un composant supplémentaire destiné à être utilisé avec le dispositif médical contenu dans la poche centrale (312).

10. Emballage scellé (2) selon la revendication 8 ou 9, dans lequel la partie centrale (12) comprend des parois latérales (38) qui supportent le dispositif (4) et une paroi inférieure (40) qui est espacée du dispositif (2).

11. Emballage scellé (2 ; 102 ; 202 ; 302) selon l'une quelconque des revendications précédentes, dans lequel la cavité (6 ; 106 ; 206 ; 306) a un profil extérieur sous le dispositif (4 ; 160, 170 ; 260, 270) qui est mis en forme de telle sorte que, lorsque l'emballage (2 ; 102 ; 202 ; 302) repose sur une surface horizontale, le centre de gravité prédéfini du dispositif (4 ; 160, 170 ; 260, 270) est situé en avant du point d'appui (32 ; 132 ; 232).

12. Emballage scellé (2 ; 102 ; 202 ; 302) selon l'une quelconque des revendications précédentes, dans lequel le dispositif médical comprend un dispositif de transfert de fluide (4) ou dans lequel le dispositif médical est constitué d'un moyeu d'aiguille (160 ; 260), portant une aiguille, et d'un capuchon d'aiguille (170 ; 270).

13. Emballage scellé (2 ; 102 ; 202 ; 302) selon l'une quelconque des revendications précédentes, comprenant en outre un support (60) agencé à l'extérieur de l'emballage (2 ; 102 ; 202 ; 302) pour déformer la cavité (6 ; 106 ; 206 ; 306) de manière à empêcher sensiblement le dispositif (4 ; 160, 170 ; 260, 270) de pivoter vers l'avant jusque dans une position stable à l'intérieur de l'emballage (2 ; 102 ; 202 ; 302).

14. Procédé de scellement d'un emballage (2 ; 102 ; 202 ; 302) contenant un dispositif médical (4 ; 160, 170 ; 260, 270) ayant un centre de gravité prédéfini, l'emballage (2 ; 102 ; 202 ; 302) comprenant :
une cavité (6 ; 106 ; 206 ; 306) contenant le dispositif médical (4 ; 160, 170 ; 260, 270) ;
un point d'appui (32 ; 132 ; 232) dans la cavité, défini comme un point de contact entre le dispositif (4 ; 160, 170 ; 260, 270) et une paroi de la cavité (6 ; 106 ; 206 ; 306), le point d'appui (32 ; 132 ; 232) étant positionné de telle sorte que le centre de gravité prédéfini du dispositif (4 ; 160, 170 ; 260, 270) est dans une direction vers l'avant par rapport au point d'appui (32 ; 132 ; 232) de telle manière que le dispositif (4 ; 160, 170 ; 260, 270) a tendance à pivoter autour du point d'appui (32 ; 132 ; 232) jusque dans une position stable ; et
une poche avant (10 ; 110 ; 210 ; 310) formée dans la cavité (6 ; 106 ; 206 ; 306) qui s'étend dans ladite direction vers l'avant et au-dessous du point d'appui (32 ; 132 ; 232) pour recevoir une extrémité avant du dispositif (4 ; 160, 170 ; 260, 270) lorsque le dispositif (4 ; 160, 170 ; 260, 270) est libre de pivoter jusque dans une position stable,
le procédé comprenant les étapes consistant à :
déformer une ou plusieurs parois de la cavité (6 ; 106 ; 206 ; 306) de telle sorte que le dispositif médical (4 ; 160, 170 ; 260, 270) contenu dans la cavité (6 ; 106 ; 206 ; 306) soit supporté sur le point d'appui (32 ; 132 ; 232) dans une position instable à l'intérieur de l'emballage (2 ; 102 ; 202 ; 302) ; et
appliquer un dos amovible (8 ; 108 ; 208 ; 308) pour sceller la cavité (6 ; 106 ; 206 ; 306).

15. Procédé selon la revendication 14, comprenant la déformation d'une ou de plusieurs parois de la poche avant (10 ; 110 ; 210 ; 310).
